# EUROPÄISCHE PATENTANMELDUNG

(11) **EP 3 103 500 A1**
(43) Veröffentlichungstag der Anmeldung: **14.12.2016**
(21) Anmeldenummer: 15001736.6
(22) Anmeldetag: 11.06.2015
(51) Int. Cl.: A61M 25/00, A61M 1/00, A61M 25/04, A61M 25/10, A61M 27/00

(54) **OFFENPORIGER BALLONKATHETER**

(71) Anmelder: Lohmann & Rauscher GmbH, 2525 Schönau/Triesting (AT)
(72) Erfinder: Loske, Gunnar, 22926 Ahrensburg (DE)
(74) Vertreter: Leinweber & Zimmermann

(57) **Zusammenfassung**

Katheter (20), insbesondere einen Ballonkatheter, mit einem Fluidleitelement (2) wie etwa einem Drainageschlauch mit einem Innenlumen (2g) zum Abführen von Körperflüssigkeit und mit einem Expansionselement wie etwa einem Ballon (1) mit veränderlichem Querschnitt zum Fixieren des Katheters in einem Körperhohlraum. Das Fluidleitelement (2) weist einen Wandabschnitt (2c) aus einem offenporigen Material (3) auf, das mit dem Innenlumen (2g) in Fluidverbindung steht und durch das die Körperflüssigkeit durch Anlage eines Unterdrucks an ein proximales Ende des Fluidleitelements (2) in das Innenlumen (2g) saugbar ist. Ferner ein Katheter-System mit Katheter (20) und einem daran angeschlossenen vakuumerzeugenden System.

## Beschreibung

Die Erfindung betrifft einen Katheter, insbesondere einen Ballonkatheter, mit einem Fluidleitelement mit einem Innenlumen zum Abführen von Körperflüssigkeiten und/oder Gasen aus dem Körperinneren und mit einem Expansionselement mit veränderlichem Querschnitt zum Halten bzw. Fixieren des Katheterabschnitts in einem Körperhohlraum. Das Fluidleitelement kann ein Schlauchelement wie etwa einen Drainageschlauch, und das Expansionselement kann einen Ballon aufweisen.

Derartige Katheter werden u.a. als Blasenkatheter verwendet, die entweder über die Harnröhre (transurethral) oder die Bauchdecke (suprapubisch) in die Harnblase eingebracht werden und der Harnableitung dienen. Insbesondere wenn der Blasenkatheter als Dauerkatheter verwendet werden soll, ist es bekannt, in der Nähe des distalen Endes (des im Körperinneren anzuordnenden Endes) des Katheters ein Expansionselement wie etwa einen aufblasbaren Ballon zur Verhinderung der Dislokation des Katheters anzuordnen, dessen Volumen veränderlich ist. Wenn der Katheter korrekt platziert ist und sich der den Ballon aufweisende Katheterabschnitt in einem Körperhohlraum wie etwa der Harnblase befindet, wird das Ballonvolumen derart vergrößert, dass sich der Ballon in dem Körperhohlraum verankert. Der Katheter ist damit selbsthaltend.

Defekte der Harnblase, des Nierenbeckens und der harnableitenden Organe führen zum Urinaustritt. Sie treten beispielsweise als Folge von postoperativen Komplikationen an diesen Organen auf. Sie können operativ verschlossen werden. Leckagen der Harnröhre, Harnleiter, aber auch von Harnblase oder Nierenbecken werden auch konservativ mit Hilfe der passiven, der Schwerkraft folgenden Urinableitung behandelt. Zur Harnableitung werden transurethrale, suprapubische Katheter oder Nierenfisteln eingebracht und hierüber der Urin in Beuteln abgeleitet. Der Urin soll dem geringsten Widerstand und der Schwerkraft folgend direkt in die Katheter geleitet werden und nicht den Weg über die Leckage nehmen. Leckagen der Harnleiter können auch über kleinlumige Schienungskatheter, welche den Defekt überbrücken, vom Nierenbecken bis ins die Harnblase oder darüber hinaus durch die Urethra hindurchgeleitet werden. Diese Katheter sind an ihrem Ende zum Teil schweineschwanzähnlich gekringelt.

Die beschriebenen Ableitkatheter oder Sonden sind in einem distalen Endabschnitt mit Perforationsöffnungen versehen. Über diese Perforationsöffnungen läuft der Urin bzw. eine andere aus dem Körper abzuleitende Körperflüssigkeit unter Schwerkraftwirkung in das Innenlumen des Drainageschlauchs ein und wird über den Drainageschlauch nach draußen abgeführt.

Aus der Druckschrift WO 2013/029622 ist es bekannt, einen Unterdruck an ein Fluidleitelement anzulegen, um eine Urindrainage zu beschleunigen. Es hat sich allerdings herausgestellt, dass durch die Anlage der Außenwand des Fluidleitelements an einer Körperinnenwand eine beschleunigte Drainage erschwert werden kann. Dieser Nachteil wird durch die Erfindung beseitigt und zahlreiche neue Möglichkeiten der Unterdruckbehandlung zur Wundbehandlung werden beschrieben.

In Anbetracht der beschriebenen Probleme ist es die Aufgabe der vorliegenden Erfindung, einen mittels eines Expansionselements im Körper verankerbaren Katheter dahingehend weiterzubilden, dass er zur schnellen und zuverlässigen Drainage von Körperflüssigkeiten durch Anlage von Unterdruck an den Katheter einsetzbar ist.

Diese Aufgabe wird durch einen Katheter, insbesondere einen Ballonkatheter, gemäß Patentanspruch 1 gelöst. Vorteilhafte Weiterbildungen der Erfindung sind in den abhängigen Ansprüchen beschrieben. Der erfindungsgemäße Katheter zeichnet sich dadurch aus, dass das Fluidleitelement einen Wandabschnitt aus einem offenporigen Material aufweist, das mit dem Innenlumen in Fluidverbindung steht. Durch das offenporige Material hindurch ist die Körperflüssigkeit durch Anlage eines Unterdrucks an ein proximales Ende (das dem Körper abgewandte Ende) des Fluidleitelements in das Innenlumen einsaugbar. Vorzugsweise ist ein distaler Endabschnitt des Fluidleitelements von dem offenporigen Material umgeben. Damit stellt die Erfindung einen offenporigen Ballonkatheter bereit, an welchen ein Unterdruck angelegt werden kann.

Die Erfindung geht auf die Erkenntnis zurück, dass die Perforationsöffnungen bei herkömmlichen Drainagekathetern leicht verstopfen, so dass keine permanente Fluidleitung über die Drainageleitung gewährleistet ist. Ferner kann sich der Drainageschlauch herkömmlicher Kathetersysteme mit Perforationsöffnungen an das Körpergewebe ansaugen, wenn an das Schlauchsystem ein Unterdruck angelegt wird. Durch das Ansaugen an das Körpergewebe werden die Perforationsöffnungen verschlossen, so dass keine Fluidleitung in das Innenlumen mehr stattfinden kann. Demgegenüber schlägt die Erfindung vor, sich selbst verankernde Katheter wie etwa Ballonkatheter als offenporige Drainage auszurüsten, so dass an die Katheter mit Hilfe von unterdruckerzeugenden Systemen ein Unterdruck angelegt werden kann. Aufgrund seiner Offenporigkeit wird ein Ansaugen des dem Körpergewebe zugewandten Wandabschnitts erschwert. Ferner verhindert die offenporige Konstruktion eine Verstopfung der Drainage und gewährleistet eine permanente Fluidleitung der Drainage. Flüssigkeiten wie z.B. Urin können somit problemlos aktiv mit Unterdruck abgeleitet werden. Mit der Erfindung wird eine schnelle und zuverlässige Ableitung von Sekreten unter Sog ermöglicht, die alle Vorteile der passiven Ableitkatheter beibehält (Platzierung durch Punktion, Platzierung durch sämtliche natürliche Körperöffnungen wie etwa Anus, Mund, Nase, Harnröhre etc., Schlauchform der gesamten Drainage, Dislokationsschutz durch Integration von Expansionselement).

Die Unterdrucktherapie (negative pressure therapy, Vakuumtherapie, low pressure therapy) hat sich als ein Behandlungsverfahren zur Therapie von äußeren infizierten Wunden etabliert. Ein offenporiges Fluidsammelmittel (bspw. Gaze oder Polyurethanschaum) wird in die Wunde eingelegt, mit einer Folie okkludiert und ein Vakuum an die Wunde angelegt. Der Verband wird in regelmäßigen Abständen gewechselt. Die Wunde reinigt sich, Wundödem- und sekrete werden abgeleitet. Es bildet sich eine stabile Wundfläche mit Granulationsgewebe, welches zur vollständigen Wundheilung führt. Es konnte bereits gezeigt werden, dass das Prinzip der Wundheilung äußerer Wunden mittels der Unterdrucktherapie in gleicher Weise intrakorporal angewendet werden kann. Mit Endoskopen lässt sich das offenporige Verbandmaterial, an welches ein vakuumerzeugendes System angeschlossen werden kann, intrakorporal über natürliche oder nicht natürliche Körperöffnungen in Körperhöhlen einlegen. Typische Behandlungsindikationen sind Leckagen am Gastrointestinaltrakt, insbesondere im Rectum und Ösophagus. Die Behandlungen werden intracorporal intracavitär in einer Wundhöhle und intraluminal direkt in Darmlumina angewandt. Desweiteren findet die Unterdrucktherapie bereits Anwendung bei Bauchfellentzündungen. Eine doppellagige Drainagefolie (Suprasorb® CNP Drainage) mit offenporigen Drainageeigenschaften kann hierzu direkt auf das Intestinum aufgelegt werden. Die Weiterleitung des Sogs an die Drainagefolie wird unter der Okklusionsfolie über offenporige Polyurethanschäume oder Gaze vorgenommen.

Bislang bereitete die Anwendung der Unterdrucktherapie bei Leckagen an den harnableitenden Organen, an der Harnblase oder dem Nierenbeckenkelchsystem Probleme. Es hat sich nun herausgestellt, dass ein erfindungsgemäßer Ballonkatheter mit Drainageschlauch infolge der ein Ansaugen an das Gewebe verhindernden und eine Saugfähigkeit permanent aufrechterhaltenden Offenporigkeit in besonders vorteilhafter Weise für intrakorporale Anwendungen der Unterdrucktherapie, insbesondere im Bereich von Körperhohlräumen wie etwa der Harnblase einsetzbar ist.

Die Erfindung kann am menschlichen und am tierischen Körper eingesetzt werden. Durch den Unterdruck können aktiv Flüssigkeiten und Gase abgeleitet werden. Insbesondere kann Urin abgeleitet werden. Es können alle liquiden interkorporalen Flüssigkeiten wie Serome, Lymphe, Hämatome, Abszeße, Ascites, Galle, Eiter, Blut, Magensaft, Dünndarmsekret, Pankreassekret, Pleuraergüße, Lymphe sowie Gase u.a. abgeleitet werden.

Das Fluidleit- und Fluidkommunikationselement des erfindungsgemäßen Katheters weist vorzugsweise einen unterdruckstabilen Schlauch auf, der auch unter der Anlage eines Unterdrucks nicht kollabiert. Im Schlauch fließen die abgeleiteten Gase und Flüssigkeiten. Der Schlauch hat mindestens ein fluidleitendes Innenlumen. Am proximalen Ende kann der Schlauch über ein Verbindungselement fluid leitend mit einem unterdruckerzeugenden System wie etwa einem Pumpsystem verbunden werden, welches mit einem Sekretauffangbehälter, in welchem das abgeleitete Sekret aufgefangen wird, ausgerüstet ist. Der Wandabschnitt aus offenporigem Material ist vorzugsweise in einem distalen Endabschnitt des Schlauchs angeordnet. Insbesondere kann eine das Innenlumen geschlossen umlaufende Schlauchwand an ihrem distalen Ende in den offenporigen Wandabschnitt übergehen.

Das offenporige Material weist vorzugsweise eine Vielzahl von untereinander fluidleitend kommunizierenden Poren auf, so dass die einzelnen Durchlässe zu dem Innenlumen auch untereinander in Fluidverbindung stehen. Die Poren können wie bei einem offenporigen Polyurethanschaum dicht an dicht stehen. Sie können auch voneinander beabstandet sein. Unter einem Unterdruck bleibt die offenporige Fluidleitfähigkeit für Flüssigkeiten und Gase bestehen. Der offenporige Wandabschnitt ist fluidleitend mit dem Innenlumen des Fluidleitelement bzw. des Schlauchs verbunden. Im Unterschied zu den seitlichen Perforationsöffnungen eines herkömmlichen Drainageschlauches, welche sich unter einem Unterdruck an die Wand einer Körperhöhle oder eines Organ ansaugen und verstopfen, so dass keine Fluidleitung mehr stattfindet, bleibt bei einem offenporig ausgerüsteten Drainageschlauch die Fluidleitung über die multipel miteinander kommunizierenden Porenöffnungen auch unter einem Unterdruck bestehen. So ist es bspw. bei der suprapubischen Anlage eines derart eingerichteten Wandabschnitts an der Harnblase möglich, dauerhaft permanent Urin aus der Harnblase aktiv abzuleiten. Die Harnblase kollabiert um das den offenporigen Wandabschnitt aufweisende distale Katheterende teilweise oder vollständig. Auf diese Art und Weise wird bei einer Leckage der Harnblase verhindert, dass Urin in einen Defekt der Harnblase eintritt. Unter der Dauerableitung kann der Harnblasendefekt ausheilen.

Die Poren haben vorzugsweise einen Öffnungsdurchmesser zwischen 100 µm und 2000 µm, insbesondere zwischen 100 µm und 500 µm. Sie können unmittelbar aneinander angrenzend oder beabstandet voneinander angeordnet sein. Der mittlere Abstand zweier nächst benachbarter Poren beträgt vorzugsweise zwischen 100 µm und 5000 µm, insbesondere zwischen 100 µm und 1000 µm.

In Sonderformen des erfindungsgemäßen Katheters ist die Beibehaltung des Prinzips der Offenporigkeit durch miteinander kommunizierende Schlitze in und/oder auf der Wand des Fluidleitelements erhalten. Die Beschreibung von Poren umschreibt nicht notwendigerweise eine kreisförmige oder runde Öffnung eines kommunizierenden Volumens. Poren können auch schlitzförmig, länglich, ellipsoid, schlauchförmig etc. sein.

Die Offenporigkeit im Bereich des distalen Endes des Katheters kann auf verschiedene Weise erreicht werden. Als hinsichtlich der Fluidabführeigenschaften besonders vorteilhaft hat sich ein offenporiger Schaum wie etwa ein offenporiger Polyurethanschaum oder eine offenporige einlagige, doppellagige oder mehrlagige Folie wie etwa die eingangs erwähnte doppellagige Drainagefolie erwiesen.

Bei einer ersten bevorzugten Ausführungsform der Erfindung ist das Innenlumen in einem distalen Endabschnitt des Fluidleitelements von einer (Perforations-)Öffnungen aufweisenden Wand wie etwa einer Schlauchwand bewandet. Diese Wandöffnungen können von dem offenporigen Material abgedeckt sein. Fluidleitung besteht von der Außenfläche des offenporigen Materials, ins Innere des Materials bis zu dem Innenlumen des Fluidleitelements.

Bei einer alternativen Ausführungsform ist das Innenlumen zumindest abschnittsweise unmittelbar von dem offenporigen Material bewandet. Dabei kann das offenporige Material in einem distalen Endabschnitt eines Drainageschlauchs dessen umlaufende Wand ausbilden und/oder sich an ein distales Ende einer nicht-offenporigen Schlauchwand anschließen. Wenn die Wand des Fluidleitelements zumindest abschnittsweise schon in sich offenporig konstruiert ist, ist sie mit unzähligen miteinander kommunizierenden Poren versehen, so dass die Fluidleitung von der Außenseite der Wand durch die Wand hindurch hin zu dem Innenlumen und/oder innerhalb und entlang der Wand besteht.

Das offenporige Material ist vorzugsweise einseitig offenporig, wobei die offenporige Seite zur Anlage an einem Körpergewebe nach außen gewandt ist und die der offenporigen Seite entgegengesetzte innere Seite dem Innenlumen zugewandt ist und Durchlässe aufweisen kann.

Das einseitig offenporige Material kann ein einseitig offenporiger Polyurethanschaum und/oder eine einseitig offenporige Folie sein. Die offenporige Seite der Folie bzw. des Schaums liegen außen, die geschlossene Seite nach innen zum Drainageschlauch bzw. zum Innenlumen. Über Öffnungen in der Schlauchwand kann die geschlossene Seite perforiert sein, so dass eine Fluidleitung von der Außenfläche des einseitig offenporigen Materials, ins Innere des Materials bis zum Innenlumen des Fluidleitelements besteht. Das einseitig offenporige Material drainiert die Körperflüssigkeit entlang und innerhalb der Wand des Fluidleitelements. Das einseitig offenporige Material kann ggf. über gesonderte Saugkanäle drainiert werden. Das Fluidleitelement kann auch zwei oder mehr Innenlumina aufweisen, die zur Drainage des offenporigen Materials genutzt werden.

Bei bestimmten Ausführungsformen ist der erfindungsgemäße Katheter so konstruiert, dass das Fluidleitelement in einem oder mehreren entlang der Katheter-Hauptachse verlaufenden Drainageabschnitten vollständig aus offenporigem Material besteht und kein entlang der Katheter-Hauptachse verlaufendes Innenlumen nach Art eines Schlauchs oder zentral verlaufenden Kanals vorgesehen ist. Zumindest ein rein aus offenporigem Material bestehender erster Drainageabschnitt kann am distalen Ende des Katheters vorgesehen sein und/oder zumindest ein rein aus offenporigem Material bestehender zweiter Drainageabschnitt kann in einem mittleren Katheterabschnitt proximal und/oder distal des Expansionselements vorgesehen sein. An das proximale und/oder an das distale Ende zumindest eines offenporigen Drainageabschnitts kann sich ein Schlauchabschnitt mit einem zentralen Innenlumen anschließen, das zum Abführen bzw. zum Weiterleiten von Körperflüssigkeiten und/oder Gasen eingerichtet ist. Mit anderen Worten weist der erfindungsgemäße Katheter nicht notwendigerweise in allen Schnittebenen einen Drainageschlauch mit einem Innenlumen auf.

Nach Anlage des Sogs am proximalen Ende des Innenlumens kann die Fluidleitung innerhalb der fluidleitenden offenporigen Drainageabschnitte stattfinden, Insbesondere soll diese Sonderform der Drainage mit einem Führungsdraht ausgerüstet werden können, der in das Innenlumen des Schlauches und durch den offenporigen Drainageabschnitt hindurchgeführt werden kann. Diese Katheterform lässt sich mit einem sehr geringen Außendurchmesser herstellen, wobei die fluidleitenden Eigenschaften weiterhin erhalten bleiben. Sie sind besonders auch für die Platzierung in kleine Lumina geeignet (Harnleiter, Gallenwege, Pankreaswege, Fisteln).

Der offenporige Wandabschnitt kann in Zug- und Schubfestigkeit verstärkt werden, indem bspw. in Längsrichtung des Katheters verlaufende verstärkende Fäden, Drähte o.dgl. in eine Wand des Fluidleitelements in diesen Abschnitten und/oder proximal und/oder distal in eine Wand des Katheters eingearbeitet sind. Die Platzierung ist unter radiologischer Kontrolle möglich. Die Platzierung ist in den Arbeitskanal von Endoskopen möglich. Der Außendurchmesser des Fluidleitelements beträgt bei dieser Ausführungsform insbesondere von 1 - 5 mm, und/oder die Katheterlänge kann zwischen 10 cm und 150 cm betragen.

In einigen Ausführungsformen kann der erfindungsgemäße Katheter am distalen Ende schweineschwanzartig gekrümmt sein, um sich mit der Krümmung in einem Hohlraum (beispielsweise einer Abszesshöhle oder einem Hohlorgan) zu verankern.

Die Drainage kann auch an beiden Enden schweineschwanzartig gekrümmt sein. Sie kann sich in diesem Fall sowohl mit ihrem distalen als auch mit ihrem proximalen Ende in einem Hohlraum verankern (z.B. als Harnleiterdrainage in Nierenbecken und Harnblase). Eine solche Sonderform kann auch als passive Drainage ohne die Anwendung einer Unterdruckanlage genutzt werden. Der Vorteil dieser erfindungsgemäßen Ausführungsform besteht darin, dass die Fluidleitung in allen Abschnitten des offenporigen Wandabschnitts des Katheters erfolgen kann, und die Flüssigkeit über die gesamte Oberfläche des offenporigen Wandabschnitts gesammelt werden kann.

Falls das offenporige Material eine oder mehrere Folien aufweist, sind diese vorzugsweise nichttransparent, insbesondere farbig oder gefärbt ausgebildet. Die gefärbten Folien dienen dem Sicherheitsaspekt bei der endoskopischen Kontrolle von inneren Wundflächen. Sollte sich eine Folie so fest an einer inneren Wunde festsaugen, dass sie ganz oder teilweise bei Entfernungsmanöver von dem Fluidleitelement abreißt, kann das Fremdmaterial bei der transparenten Ausführungsform schlecht von dem Gewebe unterschieden werden. Bei einer farbigen Ausführung kann das Fremdmaterial leicht endoskopisch entdeckt werden. Vorteilhaft ist die Folie auch röntgendicht. Alternativ kann jedoch auch eine transparente Folie verwendet werden.

Der Wandabschnitt aus offenporigem Material ist vorzugsweise in einem distalen Endabschnitt des Fluidleitelements angeordnet und kann dort das Innenlumen zumindest teilweise und vorzugsweise vollständig umlaufen und/oder distal begrenzen. Der Wandabschnitt bildet also zumindest abschnittsweise eine äußere Schicht der Seitenwand eines distalen Katheterabschnitts aus. Alternativ oder zusätzlich kann der Wandabschnitt aus offenporigem Material auch das distale Ende des Katheters, also dessen vordere Frontwand ausbilden.

Das Expansionselement weist vorzugsweise einen Ballon mit einer elastisch dehnbaren Wand auf, die zumindest teilweise ein Füllvolumen des Ballons bewandet. Durch Füllen des Füllvolumens des Ballons wird dieser aufgeblasen, und es vergrößert sich dessen Querschnitt, wodurch eine Verankerung des Katheters in einem Körperhohlraum auf besonders gewebeschonende Weise möglich ist. Die Ballonwand kann aus einem flexiblen dehnungsfähigen Gewebe wie etwa aus dehnbarem Kunststoff gebildet sein. Anstelle des Ballons können ausdrücklich auch andere, sich um oder an dem Drainageschlauch expandierende Verrieglungsmechanismen verwendet werden. Diese können beispielsweise aus sich schirmartig öffnenden bzw. abspreizenden und wiederverschließbaren Wandabschnitten oder aus einer Gegendruckplatte der Schlauchwand bestehen.

Es kann ein fluid- und/oder gasleitender Kanal zum Befüllen und/oder Entleeren des Füllvolumens des Ballons vorgesehen sein, der im Wesentlichen parallel zu dem Fluidleitelement verläuft. Der Kanal kann zumindest abschnittsweise im Innenlumen des Fluidleitelements, außerhalb des Innenlumens des Fluidleitelements, in einer oder anliegend an eine Seitenwand des Fluidleitelements in dessen Längsrichtung oder alternativ als separater Schlauch beabstandet von dem Fluidleitelement verlaufen.

Bei einer ersten erfindungsgemäßen Ausführungsform verläuft der Kanal in Längsrichtung des Fluidleitelements entlang dessen und insbesondere in dessen Außenwand als zusätzliches gas- und/oder wasserdichtes Lumen. Bspw. verläuft der Kanal in der Schlauchwand des Drainageschlauchs. Bei einer zweiten erfindungsgemäßen Ausführungsform ist der Kanal von dem Drainageschlauch beabstandet und außerhalb des Innenlumens gelegen. Bspw. ist der Kanal in Form eines zusätzlichen Schlauchs gebildet. Bei einer dritten erfindungsgemäßen Ausführungsform verläuft der Kanal als innerhalb des Innenlumens des Fluidleitelements angeordneter Schlauch. Wenn der Drainageschlauch mit mehr als einem fluidleitenden Innenlumen ausgerüstet ist, kann eines der Lumina als Kanal für die Füllung des Ballons und das andere zum Abführen der Körperflüssigkeit dienen.

Durch ein Ventil am proximalen Ende des Kanals kann verhindert werden, dass das eingefüllte Gas oder die Flüssigkeit entweicht, und es kann sichergestellt werden, dass der Ballon dauerhaft aufgepumpt bleibt. Das Gas bzw. die Flüssigkeit kann über das Ventil aus dem Ballon entfernt werden. Das Gas bzw. die Flüssigkeit kann nicht bzw. nur langsam aus dem Füllvolumen entweichen, so dass der Füllungszustand des Ballons nur selten (weniger als einmal am Tag) kontrolliert werden muss.

Der Ballon fasst vorzugsweise ein Volumen von 0 bis 200 ml. Der Ballon umläuft den Umfang des Fluidleitelements zumindest teilweise, vorzugsweise vollständig und/oder ist im aufgeblasenen Zustand ringförmig und/oder im Wesentlichen zylindrisch. Alternativ kann der Ballon im aufgeblasenen Zustand kugelig sein und das distale Ende des Katheters bilden.

Alternativ kann der Ballon den Katheter nur abschnittsweise und nicht vollständig circulär umschließen. Weiter alternativ kann der erfindungsgemäße Katheter mehr als ein Expansionselement wie etwa zwei oder mehrere Ballons aufweisen.

Es können ein oder mehrere weitere Kanäle zur Spülung, zur Zufuhr von Medikamenten, zur Druckmessung und/oder zur passiven Drainage vorgesehen sein. Ein solcher Spülkanal ist an seinem distalen Ende offen. Über den Spülkanal können dem zu drainierenden Cavum (Harnblase, Abszesshöhle) Flüssigkeiten oder Medikamente zugeführt werden, oder es kann gespült werden. Über zumindest einen weiteren Kanal können auch Messinstrumente bspw. zur Steuerung der vakuumerzeugenden Einheit wie zur Unterdruckmessung zugeführt oder anderweitige Messungen über das Lumen des Kanals vorgenommen werden. Der Katheter soll auch zur Saugspülbehandlung benutzt werden können. Er kann beispielweise zur Fortführung einer Wundbehandlung nach Rectumanastomoseninsuffizienz mit einer Spülbehandlung genutzt werden. Dann würde der Katheter rektal bis zum Spülort eingeführt werden, über den einen Kanal gespült und mit dem anderen Kanal das Spülsekret aktiv durch Sog oder passiv abgeleitet werden. Der ableitende Kanal ist offenporig konstruiert und kann ggf. zusätzlich mit Perforationsöffnungen am distalen Kanalende versehen sein.

Bei einer bevorzugten Ausführungsform der Erfindung breitet sich der Ballon durch Insufflation von Gas oder Flüssigkeit über den Kanal ganz oder teilweise ringförmig um das Fluidleitelement herum oder kugelig am distalen Ende des Katheters aus. Der Ballon kann als ein Teil einer Seitenwand des Fluidleitelements ausgebildet sein, wobei die Wand in diesem Abschnitt flexibel und elastisch ausdehnbar eingerichtet ist, so dass der Ballon mit Flüssigkeit und/oder Gas gefüllt werden kann.

Bei einer besonderen Ausführungsform der Erfindung ist das Füllvolumen des Ballons zumindest teilweise von einem Füllmaterial wie etwa einem offenporigen Polyurethanschaum gefüllt, das sich bei Anlegen eines Unterdrucks an das Füllvolumen zusammenzieht und das bei Aufheben des Unterdrucks eine Entfaltung des Ballons bewirkt. Diese Funktion hat den Vorteil, dass der Ballon sich mit dem Füllmaterial dem zu entlastenden Hohlraum anpassen kann. Das Füllmaterial kann verschiedene Formen haben. Beispielsweise kann ein solcher Ballon die Form einer Kugel oder eines Zylinders einnehmen. Die Kugelform soll insbesondere einen Durchmesser von 1 cm bis 10 cm haben. Die Zylinderform soll insbesondere einen Durchmesser von 1 bis 5 cm und eine Länge von 1 cm bis 20 cm haben. An den Ballon kann ein Unterdruck angelegt werden, so dass er in sich vollständig kollabiert. Diese Funktion kann die Platzierung einer Ballondrainage (z.B. beim Wechseln der Drainage) erleichtern.

Im Hinblick auf eine gute und zuverlässige Platzierung des offenporigen Materials hat es sich als zweckmäßig erwiesen, dass sich der Wandabschnitt aus dem offenporigen Material unmittelbar distal an den Ballon anschließt. Es ist aber auch möglich, dass der Ballon selbst das distale Ende des Katheters bildet und der Wandabschnitt aus dem offenporigen Material zumindest abschnittsweise außen an der elastisch dehnbaren Wand des Ballons angeordnet ist.

Bei einer bevorzugten Ausführungsform der Erfindung ist der Wandabschnitt aus offenporigem Material an einem distalen Endabschnitt des Fluidleitelements angeordnet. Alternativ oder zusätzlich ist es möglich, einen Wandabschnitt aus offenporigem Material in einem mittleren Abschnittes des Katheters vorzusehen. Das Expansionselement kann beabstandet von dem offenporigen Wandabschnitt angeordnet sein. Beispielsweise kann ein so ausgerüsteter Katheter dazu genutzt werden, um mit dem in dem mittleren Abschnitt des Katheters angeordneten offenporigen Wandabschnitt eine Harnleiternaht oder eine Harnleiterundichtigkeit abzudichten, während das Expansionselement im Nierenbecken verankert werden kann.

Bei einer möglichen Ausführungsform der Erfindung befinden sich Wandabschnitte aus offenporigem Material distal und/oder proximal des Expansionselementes. Der bzw. die offenporige(n) Wandabschnitt(e) gehen vorzugsweise stufenlos in nicht-offenporige Wandabschnitte des Katheters über, um ein atraumatisches Einbringen zu erleichtern. Ein Katheter soll mehrere offenporige Wandabschnitte aufweisen können, die benachbart zueinander oder voneinander beabstandet angeordnet sein können.

Der Drainageschlauch des Fluidleitelements hat vorzugsweise einen Durchmesser zwischen 2 mm und 25 mm. Alternativ oder zusätzlich hat der in den Körper einführbare Teil des Katheters eine Länge zwischen 15 cm und 150 cm. Alternativ oder zusätzlich hat der Katheter im Bereich des aufgeblasenen Ballons einen Gesamtdurchmesser zwischen 5 mm und 50 mm. Der Querschnitt des Katheters ist durch Expandieren des Expansionselements etwa um den Faktor 2 oder mehr vergrößerbar.

Der offenporige Wandabschnitt hat vorzugsweise eine Länge von mehr als 1 cm und weniger als 15 cm.

Eine einfache Platzierung des Katheters im Körper ist dadurch möglich, dass dessen distales Ende geschlossen ist und/oder ein Befestigungselement wie etwa eine Öse, einen Faden, eine Kugel o.dgl. zum Anbringen eines Greif- oder Zugelements aufweisen kann. Das geschlossene distale Ende kann konisch oder spitz zulaufend ausgebildet sein, wobei das Befestigungselement an der Spitze angebracht ist. Mit solch einer Ausrüstung kann der Katheter in Pull-Through-Technik durch Zug an der Spitze in Körperhöhlen platziert werden. Mit dem geschlossenen distalen Ende kann der Drainageschlauch besonders atraumatisch in den Körper durch natürliche Körperöffnungen (z.B. Harnröhre) oder bestehende andere Öffnungen (z.B. Fistelöffnungen, suprapubisches Stoma zur Harnblase, o.dgl.) eingeführt werden. Wenn nach dem Einführen mit der Spitze des Drainageschlauchs die zu drainierende Höhle (z.B.. Harnblase oder Nierenbecken) erreicht ist, wird der Ballon gefüllt und geblockt. Durch den aufgefüllten Ballon wird die Dislokation des Katheters verhindert.

Bei der suprapubischen Platzierung eines offenporigen Ballonkatheters soll der Ballon auch dazu dienen, die Harnblase mit dem Katheter an die Bauchdecke zu ziehen. Zumindest zu Beginn der Therapie soll der Ballonkatheter mit leichtem Zug an die Bauchdecke gezogen werden. Das Heranziehen der Wand des Hohlorgans an die Bauchdecke dient insbesondere dazu, dass das Hohlorgan mit der Bauchwand sicher verklebt und ein Fistelkanal entsteht. Dies ist besonders wichtig, da in der Regel bei der Platzierung des Katheters ist das Hohlorgan gefüllt ist und nach Anlage des Vakuums kollabiert, und daher äußere und innere Punktionsstellen auseinandergleiten können. Der Zug wird aufrechterhalten, indem der Katheter auf der Haut über der cutanen Eintrittsöffnung fixiert wird. Diese Fixierung unter Zug kann mit einer chirurgischen Fixierungsnaht und/oder mit einer Gegendruckplatte vorgenommen werden, in der sich der Katheter unter Zug verklemmt. Wenn sich ein stabiler Kanal um den Katheter gebildet hat, kann der Zug gelockert oder aufgehoben werden, da ansonsten eine Druckschädigung der Blasenschleimhaut auftreten kann. In Analogie zu dieser Beschreibung der Handhabung bei der Harnblase gilt Ähnliches für die Anwendung an anderen Hohlorganen, beispielsweise bei einer percutanen Magenpunktion zur Entlastung des Magens.

Bei einer weiteren Ausführungsform der Erfindung weist der Katheter an seinem distalen Ende eine Öffnung auf, durch die ein Führungsdraht oder Führungsmandrin einführbar ist. Der Führungsdraht kann atraumatisch, am distalen Ende flexibel und weich ausgestattet sein. Mit einem derartigen Draht kann der Katheter beispielsweise in Seldingertechnik in den Körper eingeführt werden.

Der Führungsdraht oder Führungsmandrin kann an seinem distalen Ende eine Schneide oder Spitze zum Punktieren von Gewebe aufweisen. Der Drainageschlauch soll mit seinem distalen Ende bündig und stufenlos mit dem Führungsmandrin abschließen. Mit einer solchen Ausrüstung kann der Katheter in Punktionstechnik in Körperhöhlen eingebracht werden. Beispielsweise können perkutan Abszesse, suprapubisch die Harnblase, perkutan das Nierenbecken, Pleuraergüsse oder jegliche erreichbare Flüssigkeitsansammlungen punktiert werden. Die Punktion kann unter sonographischer, radiologischer, computertomographischer und/oder endoskopischer Sichtkontrolle erfolgen. Bei der suprapubischen Punktion kann der Katheter auch über das Lumen eines in der Längsachse teilbaren, am distalen Ende scharfen Punktionsrohres wie ein suprapubischer Blasenkatheter eingelegt werden.

Der erfindungsgemäße Katheter kann an seinem distalen Ende in der Längsachse gestreckt und gerade laufend sein. Für andere Anwendungen kann der Katheter an seinem distalen Ende in der Längsachse bogenförmig gekrümmt sein Für andere Anwendungen kann der Katheter an seinem distalen Ende in der Längsachse schweineschwanzigartig gekringelt sein. Auch wenn der Katheter nicht gestreckt gerade verläuft, kann er über einen Führungsdraht gestreckt gerade laufen.

Zur schnellen Ableitung von Körperflüssigkeit auch ohne das Anlegen eines Unterdrucks kann es vorteilhaft sein, das Fluidleitelement an seinem distalen Ende mit mindestens einer seitlichen Perforationsöffnung auszustatten, die nicht durch das offenporige Material abgedeckt ist. Über diese Öffnungen können Flüssigkeiten oder Gase der Kapillarwirkung oder der Schwerkraft folgend abgeleitet werden. Diese Öffnungen sind vorzugsweise distal des Wandabschnitts aus einem offenporigen Material angeordnet.

Der offenporige Abschnitt des Fluidleitelements kann stufenlos in einen schlauchförmigen Abschnitt übergehen. Der Durchmesser des Fluidleitelements kann in beiden Abschnitten im Wesentlichen gleich sein. Der Durchmesser des Fluidleitelements sollte im offenporigen Abschnitt nur unwesentlich größer oder kleiner sein als der Durchmesser des geschlossenen Schlauchabschnitts. Hierdurch soll insbesondere die leichtere Platzierbarkeit und Entfernbarkeit des Katheters, beispielsweise bei der transurethralen Platzierung gewährleistet sein. Insbesondere kann die Drainage auch operativ als postoperative Drainage in die Bauchhöhle, in einen Abszess oder thorakal platziert werden. Hier werden herkömmlicherweise bislang passive Drainagen eingesetzt. Mit der Erfindung hat man den Vorteil, eine aktive Unterdruckdrainage zu platzieren, welche sich über einen Ballon verankern und nicht dislozieren kann, an welche eine aktive Saugung angeschlossen werden kann und welche postoperativ ohne eine weitere Operation lediglich durch Zug an der entblockten Drainage entfernt werden kann.

Der erfindungsgemäße Katheter kann zur gleichzeitigen oder im Intervall stattfinden Saug-Spülung von intrakorporalen Räumen eingesetzt werden.

Der offenporige Wandabschnitt des Fluidleitelements liegt vorzugsweise distal des Ballons.

Alternativ oder zusätzlich liegt der offenporige Wandabschnitt des Fluidleitelementes proximal des Expansionselements. In diesem Fall kann das Expansionselement den distalen

Abschluss des Fluidleitelementes bilden. Ein derart ausgebildeter Katheter kann insbesondere auch als postoperative Drainage beispielsweise intraabdominell zum Abschluss der Operation platziert werden. Das Expansionselement verankert die Drainage innerlich am Platzierungsort. Die Drainage kann sowohl als passive Drainage als auch als aktive Drainage mit Anlage eines Unterdrucks angewandt werden. Ein weiteres typisches Anwendungsbeispiel für eine solche Drainage ist eine Wunddrainage oder eines Abszessdrainage.

Es ist ausdrücklich auch daran gedacht, eine den Ballon umgebende Wand zusätzlich offenporig und/oder einseitig offenporig auszugestalten, so dass auch an den ballontragenden Wandabschnitt bei entfaltetem, inkomplett entfaltetem oder nicht entfaltetem Ballon ein Unterdruck angelegt werden kann. Spezialformen der Unterdruckdrainage bestehen aus einem Katheter, welcher an seinem distalen Ende einen Ballon mit einer zur Fluidleitung geeigneten offenporigen Wand trägt, an die ein Unterdruck angelegt werden kann. Ein solcher Katheter ist mindestens zweilumig ausgerüstet, wobei das eine Lumen zur Expansion des Ballons und das andere Lumen zur Drainage des Fluids unter Anlage des Vakuums dient. Ein Vorteil dieser Spezialdrainage besteht in der atraumatischen Lage in einem Hohlorgan. Es ragen keine Schlauchenden oder Spitzen über den Ballon hinaus, welche unter dem Sog auch zu einer Druckschädigung der Organwand führen könnten. Die offenporige und/oder einseitig offenporige Umhüllung des Ballons kann fest mit der Ballonwand verbunden sein. Die offenporige und/oder einseitig offenporige Umhüllung kann auch lose wie ein Beutel oder Mantel um den Ballon liegen. Die offenporige Umhüllung ist elastisch und passt sich dem Ballon an. Der Katheter kann auch insbesondere zur operativen Drainage nach Bauchhöhleneingriffen eingesetzt werden.

Gemäß einem weiteren Gesichtspunkt betrifft die Erfindung ein Katheter-System mit einem erfindungsgemäßen Katheter und einem unterdruckerzeugenden System wie etwa einem elektronischen Pumpsystem, das zum Erzeugen eines Sogs in dem Innenlumen des Fluidleitelements an das proximale Ende des Fluidleitelements anschließbar ist.

Zwischen das unterdruckerzeugende System und das Fluidleitelement kann mindestens ein unterdruckstabiler Fluidauffangbehälter zum Auffangen der Körperflüssigkeit geschaltet sein, der vorzugsweise mit einem einen Rückfluss von Körperflüssigkeit in das Fluidleitelement verhindernden Ventil ausgestattet ist.

Das unterdruckerzeugende System sollte zur Erzeugung eines permanenten Unterdrucks zwischen etwa 10 mmHg und etwa 200 mmHg, insbesondere zwischen etwa 30 mmHg und etwa 100 mmHg, eingerichtet sein.

Wenn nur kleine Mengen an Körperflüssigkeit abgeleitet werden sollen, ist ein Behältervolumen zwischen 100 ml und 500 ml ausreichend. Es können auch größere Behälter an das Pumpsystem angeschlossen sein. Insbesondere im Falle der Urinableitung, für welche die Erfindung in besonderem Maße geeignet ist, sind auch größere Mengen an Körperflüssigkeit abzuleiten. Hier kann zwischen den Katheter und den Fluidauffangbehälter noch ein weiterer unterdruckstabiler Auffangbehälter, der mit dem Ventil ausgestattet sein kann, zwischengeschaltet werden. Hiermit lassen sich Mengen zwischen 500 ml und 2500 ml auffangen. Dies hat den Vorteil, dass die vakuumerzeugende Pumpe (Pumpe mit kleinem Behälter zur Vakuumvorerzeugung) relativ kompakt und klein gehalten werden kann. Bei Verwendung von sehr großen Auffangbehältern an der Pumpe selbst wird diese sonst sehr unhandlich und der Patient hierdurch immobil.

Die elektronische Pumpe soll vorzugsweise kleinformatig sein. Dies ist insbesondere dann möglich, wenn keine hohen Unterdrücke und kein hohes Sogvolumen/min erforderlich sind. Eine solche Pumpe soll beispielsweise zur aktiven Harnableitung aus dem Nierenbecken und/oder aus der Harnblase eingesetzt werden. Hier ist aufgrund der intrakorporalen Lage bei der transurethralen oder suprapubischen Platzierung (anders als bei der Therapie von äußeren Wunden mit Okklusionsverbänden) nicht mit Leckagen an einem Verband zu rechnen.

Die Pumpe soll bei dieser Anwendung das anfallende Urinvolumen drainieren können. Dieses liegt im Normalfall bei gesunden Erwachsenen zwischen 1,5 l und 2 l pro 24 Stunden. Abhängig von der Trinkmenge (Infusionsmenge) oder krankheitsbedingt kann die Urinmenge auch mehr als 2 l am Tag betragen. Da die Urinproduktion mit gewissen Schwankungen kontinuierlich anfällt, muss die Pumpe bei einer Urinproduktion von 2 l in 24 Stunden in der Lage sein, pro Stunde im Durchschnitt mindestens ca. 100 ml zu drainieren. Die Pumpe sollte in der Lage sein, 500 ml Fluid pro Stunde zu drainieren. Da der natürliche Urinfluss durch Kapillarleitung und muskuläre Harnleiterkontraktion in die Harnblase geleitet wird und sich bei passiven Drainagen oder Leckagen entlang der Schwerkraft drainiert, muss die Pumpe zumindest in der Lage sein, entgegengesetzt zur Schwerkraftwirkung zu drainieren. Wenn das vakuumerzeugende System auf gleichem Niveau wie die zu drainierende Harnblase liegt, reicht bereits ein Sog von wenigen cm Wassersäule zur Drainage. Liegt das vakuumerzeugende System oberhalb des zu drainierenden Orts, muss zunächst diese Höhendifferenz überbrückt werden. Analog gilt dies für den umgekehrten Fall, wenn die Pumpe unter Niveau platziert wird.

Unabhängig von der Lage der abzuleitenden Körperflüssigkeit sollte die Pumpe daher in der Lage sein, innerhalb von einer Minute mindestens einen Unterdruck von 20 mmHg an das Fluidleitelement anzulegen. Dieser Sog soll permanent an der Harnblase anliegen. Er kann durch eine Steuerung permanent nachgeregelt werden. Die Pumpe sollte für die Anwendung in der Harnblase vorzugsweise einen Unterdruck von 20 mmHg bis 100 mmHg erzeugen können.

Bei einer ersten erfindungsgemäßen Ausführungsform erzeugt die Pumpe ihr Vakuum über einen Vorbehälter. Bei einer alternativen erfindungsgemäßen Ausführungsform erzeugt die Pumpe das Vakuum ohne Vorbehälter. Die Pumpe ist vorzugsweise derart eingerichtet, dass sie mit einem Fluidauffangbehälter, der ein Volumen von 250 -2000 ml hat, fluidleitend verbunden werden kann. Wenn die Pumpe einen Vorbehälter aufweist, kann dieser in den Fluidauffangbehälter integriert sein. Eine zur Harnableitung zu verwendende Pumpe sollte in ihren Abmessungen nicht größer sein als etwa 5cmx10cmx20cm.

Die Handhabung des erfindungsgemäßen Systems kann dadurch vereinfacht werden, dass die Pumpe mit einem wiederaufladbaren Akku betrieben werden kann. Alternativ oder zusätzlich ist die Pumpe mit einem lösbaren Verriegelungsmechanismus an den Fluidauffangbehälter anschließbar. Der Transport der Pumpe wird dadurch vereinfacht, dass die Pumpe mit dem einen oder den mehreren Fluidauffangbehältern wie etwa einem Beutel- oder Kanistersystem eine Einheit darstellt. Die Pumpe kann also ein Teil eines Beutel-/Kanistersystems zur Körperfluidsammlung sein. Dazu kann die Einheit aus Fluidauffangbehälter und Pumpe mit einem Tragegriff und/oder Gurt versehen sein.

Besonders vorteilhaft ist die Handhabung dann, wenn das Sekret bzw. die Körperflüssigkeit in ein Beutelsystem abgeleitet wird. Das Beutelsystem muss den Sog aufnehmen und weiterleiten können. Dies kann erreicht werden, wenn das Beutelsystem ein Füllmaterial wie etwa ein offenporiges und/oder kompressibles Fluidsammelelement (z.B. einen offenporigen Polyurethanschaum) aufweist. Das Füllmaterial kollabiert bei dem verwendeten Lumen nicht oder nicht vollständig und hält das Lumen des Beutels zur Sekretaufnahme unter Aufrechterhaltung des Sogs offen. Der Vorteil des Beutelsystems liegt auch in dessen kleinem Volumenbedarf bei Transport und Lagerung. Durch Leersaugen des Beutelsystems vor der Anwendung mit einem Hochvakuum oder durch Kompression lassen sich sehr kleine platzsparende Verpackungseinheiten herstellen.

Durch eine Kammerung des Beutelsystems kann eine schrittweise Füllung Kammer für Kammer erfolgen. Es kann auch eine Einleitung des Urins in einen Beutelabschnitt erfolgen, der nicht mit dem Füllmaterial ausgerüstet ist.

Es ist ferner auch daran gedacht, als Fluidauffangbehälter eine Kombination aus Beutelsystem und festem Kanisterbehälter zu verwenden. Der Kanisterbehälter kann als Vorvolumen für die Pumpe fungieren.

Die Beutel können auch bei der Ableitung von Sekret bei der abdominalen Unterdrucktherapie mit Drainagefolien, bei der pleuralen Drainage oder bei der aktiven Magenentlastung eingesetzt werden. Hier fallen häufige größere Sekretlumina an.

Der Auffangbehälter wie etwa der Beutel und/oder der Kanister sind vorzugsweise mit Messmarkierungen zur Volumenbestimmung versehen, um die Sekretmenge bilanzieren zu können.

Der erfindungsgemäße Katheter kann zur Vereinfachung der Handhabung mit einer Markierung (vorzugsweise cm-Markierung) versehen sein, um seine Einlagetiefe ablesen und eine Dislokation feststellen zu können. Alternativ oder zusätzlich kann eine Röntgenmarkierung vorgesehen sein.

Es wird mit einem Unterdruck zwischen 10 und 200 mmHg, vorzugsweise mit einem Unterdruck zwischen etwa 40 und 200 mmHg, therapiert. Die Ableitung kann kontinuierlich oder intermittierend erfolgen. Bei der Urinableitung werden insbesondere Unterdrücke zwischen 20 und 100 mmHg angewandt. Die Pumpe kann mit einer Steuerungs- und Alarmfunktion zur Drucksteuerung ausgerüstet sein. Dadurch können Drainageblockaden-, Drainageleckagen, der Kanister- und/oder der Beutelfüllungszustand registriert und an die Steuereinheit gemeldet werden.

Es folgen exemplarisch zwei typische Anwendungsbeispiele für die Erfindung.

### Beispiel 1:

Bei einer Operation am Enddarm kommt es zu einer Blasenverletzung an der Blasenhinterwand, die übernäht wird. Die Harnblase wird intraoperativ mit einem herkömmlichen transurethralen Katheter und suprapubischen Katheter zur Harnableitung versorgt. Im postoperativen Verlauf tritt über die Operationsdrainage Urin aus. Da die Harnableitung über die bereits einliegenden, passiv ableitenden Katheter nicht ausreichend war, hatte sich der Urin über den nicht vollständig verschlossenen Defekt in die Bauchhöhle entleert und Anschluss an die Operationsdrainage gefunden. In der Erwartung, den Urinaustritt durch die direkte Urinableitung von den Nieren verhindern zu können und die Harnblase trocken zu legen, werden transurethral über beide Harnleiter in beide Nierenbecken Harnleiterschienenkatheter eingelegt. Auch hiernach gelingt die Harnableitung über die passiven Drainagen nicht, es persistiert die Urinableitung in die Operationsdrainage.

Jetzt folgt die Behandlung mit der Erfindung: Der suprapubische Katheter wird in Seidingertechnik gegen einen erfindungsgemäßen Katheter in Form einer offenporigen Ballondrainage gewechselt, der Ballon geblockt und mit einer elektronischen Pumpe ein kontinuierlicher permanenter Sog von 80 mmHg an das offenporige distale Ende der Drainage angelegt. Unmittelbar nach der Anlage des Unterdrucks kollabiert die Harnblase und der gesamte noch in der Harnblase verbliebene Urin wird vollständig abgesaugt. In der Folge sistiert die Urinsekretion über die Operationsdrainage und den transurethralen Katheter. Anders als bei der Verwendung herkömmlicher Katheter kann jetzt bei der kollabierten Harnblase die nahezu vollständige Urinproduktion über die beiden transurethral ausgeleiteten Nierenfisteln abgeleitet werden. Nach einigen Tagen wird die offenporige Ballondrainage einmalig in Seldingertechnik über einen Führungsdraht gewechselt. Hierbei wird endoskopisch die Harnblase und die innere Wunde inspiziert und der innere Wundheilungsverlauf kontrolliert. Nach 18 Tagen Urinableitung kann nach Abheilung des Harnblasendefektes die offenporige Drainagentherapie beendet werden. Die Abheilung des Defektes wurde zuvor sowohl radiologisch als auch endoskopisch bestätigt. Alle transurethralen Katheter und die Operationsdrainage werden entfernt. Die Unterdruckanlage an die offenporige Drainage wird beendet und die Drainage noch zur passiven Urinableitung für einige Tage zum Blasentraining in der Harnblase belassen. Nach erfolgreichem Blasentraining unter passiver Harnableitung kann der erfindungsgemäße Katheter als letzte Drainage entfernt werden.

### Beispiel 2:

Eine Patientin hat ein postoperatives Serom entwickelt, welches durch eine sonographisch gestütze Punktion gut erreicht werden kann. Nach sonographischer Lokalisation und Lokalanästhesie erfolgt die Punktion mit einem erfindungsgemäßen Katheter in Form einer Ballondrainage, welche mit einem scharfen Führungsmandrin ausgerüstet ist. Die Spitze des Katheters wird in der Seromhöhle platziert, der Ballon geblockt und der Katheter in der Höhle fixiert. An das offenporige distale Ende wird mit einem elektronischen Pumpensystem ein Unterdruck von 80 mmHg mit kontinuierlichem Sog angelegt. Die Seromhöhle wird vollständig aktiv dauerhaft abgesaugt, die Höhle kollabiert um die Drainage. Der Sog wird für insgesamt 3 Tage aufrechterhalten. Die sonographische Kontrolle bestätigt die vollständige Drainage des Seroms und die Drainage kann entfernt werden.

In der nun folgenden Beschreibung wird die Erfindung unter Bezugnahme auf die beigefügten Zeichnungen beispielhaft beschrieben. Die Erfindung ist nicht auf die in den Zeichnungen dargestellten Ausführungsformen beschränkt. In den Zeichnungen zeigt:
- Fig. 1a: eine erste Ausführungsform eines erfindungsgemäßen Ballonkatheters in einer Seitenansicht,
- Fig. 1b: die in Fig. 1a dargestellte erste Ausführungsform in einer Längsschnittansicht,
- Fig. 1c: eine zweite Ausführungsform eines erfindungsgemäßen Ballonkatheters in einer Seitenansicht,
- Fig. 1d: die in Fig. 1c dargestellte zweite Ausführungsform in einer Längsschnittansicht,
- Fig. 1e: eine dritte Ausführungsform eines erfindungsgemäßen Ballonkatheters in einer Seitenansicht,
- Fig. 1f: die in Fig. 1e dargestellte dritte Ausführungsform in einer Längsschnittansicht,
- Fig. 2a: eine vierte Ausführungsform eines erfindungsgemäßen Ballonkatheters in einer Seitenansicht,
- Fig. 2b: die in Fig. 2a dargestellte vierte Ausführungsform in einer Längsschnittansicht,
- Fig. 3a: eine fünfte Ausführungsform eines erfindungsgemäßen Ballonkatheters in einer Seitenansicht,
- Fig. 3b: die in Fig. 3a dargestellte fünfte Ausführungsform in einer Längsschnittansicht,
- Fig. 3c: einen Wandabschnitt eines Fluidleitelements einer sechsten Ausführungsform eines erfindungsgemäßen Ballonkatheters in einer Seitenansicht,
- Fig. 3d: die Detaildarstellung von Fig. 3c in einer Längsschnittansicht,
- Fig. 3e: einen Wandabschnitt eines Fluidleitelements einer siebten Ausführungsform eines erfindungsgemäßen Ballonkatheters in einer Seitenansicht,
- Fig. 3f: die in Fig. 3e dargestellte Ausführungsform in einer Längsschnittansicht,
- Fig. 3g: eine achte Ausführungsform eines erfindungsmäßigen Ballonkatheters in einer Seitenansicht,
- Fig. 3h: die in Fig. 3g dargestellte Ausführungsform in einer Längsschnittansicht,
- Fig. 4a: eine neunte Ausführungsform eines erfindungsgemäßen Ballonkatheters in einer Seitenansicht,
- Fig. 4b: die in Fig. 4a dargestellte neunte Ausführungsform in einer Längsschnittansicht, wobei der Ballon nicht aufgeblasen ist,
- Fig. 4c: die in Fig. 4a dargestellte neunte Ausführungsform in einer Längsschnittansicht, wobei der Ballon aufgeblasen ist,
- Fig. 5a: eine zehnte Ausführungsform eines erfindungsgemäßen Ballonkatheters in einer Längsschnittansicht, wobei der Ballon nicht aufgeblasen ist,
- Fig. 5b: die in Fig. 5a dargestellte zehnte Ausführungsform in einer Längsschnittansicht, wobei der Ballon aufgeblasen ist,
- Fig. 6a/b: eine erste Ausführungsform eines erfindungsgemäßen Katheter-Systems in einer Seitenansicht und in einer Schnittansicht,
- Fig. 7a/b: eine zweite Ausführungsform eines erfindungsgemäßen Katheter-Systems in einer Seitenansicht und in einer Schnittansicht,
- Fig. 8a/b: eine dritte Ausführungsform eines erfindungsgemäßen Katheter-Systems in einer Seitenansicht und in einer Schnittansicht, und
- Fig. 9a/b: eine vierte Ausführungsform eines erfindungsgemäßen Katheter-Systems in einer Seitenansicht und in einer Schnittansicht.

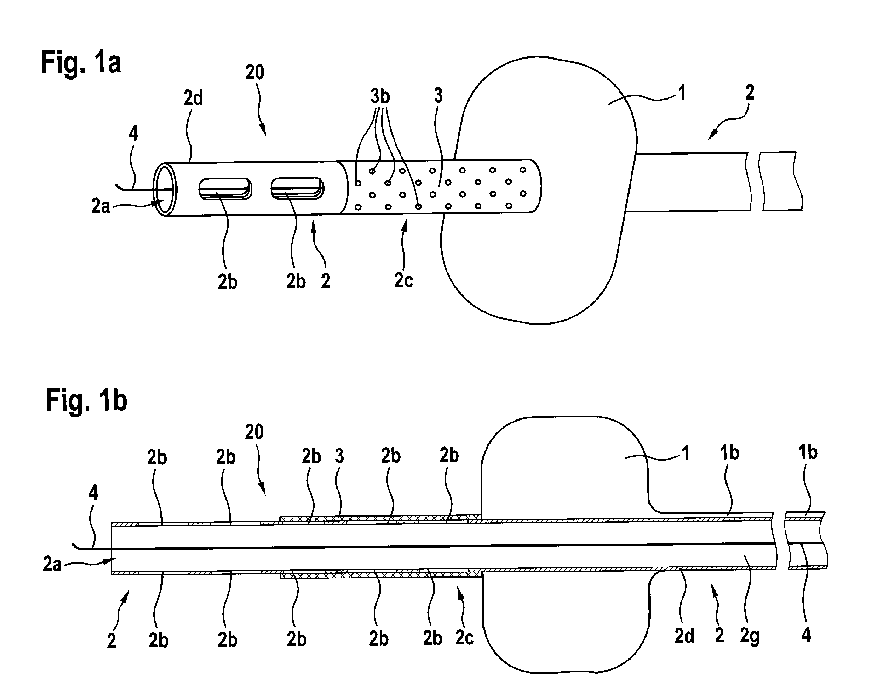

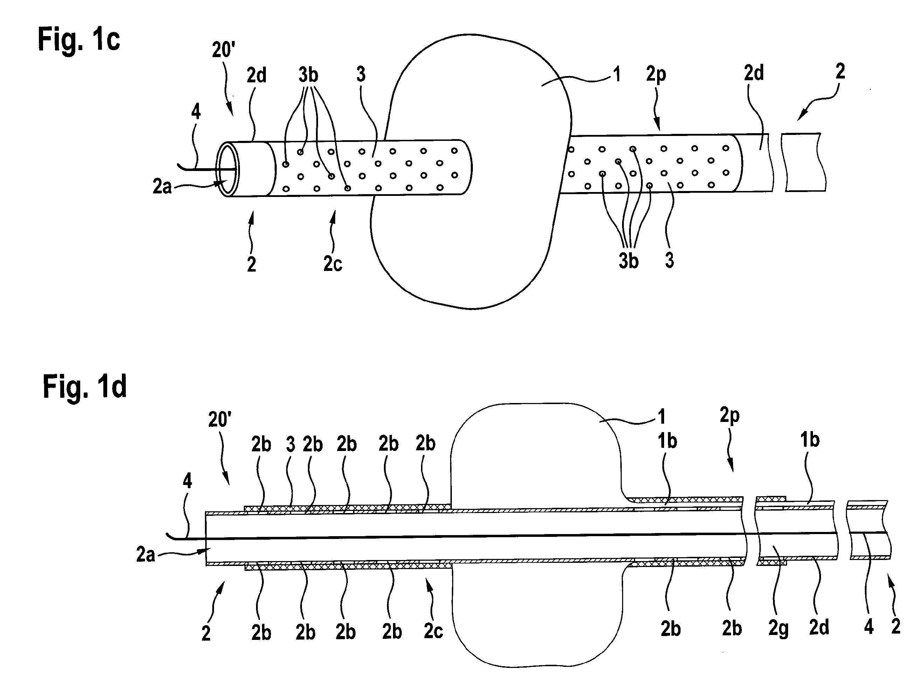

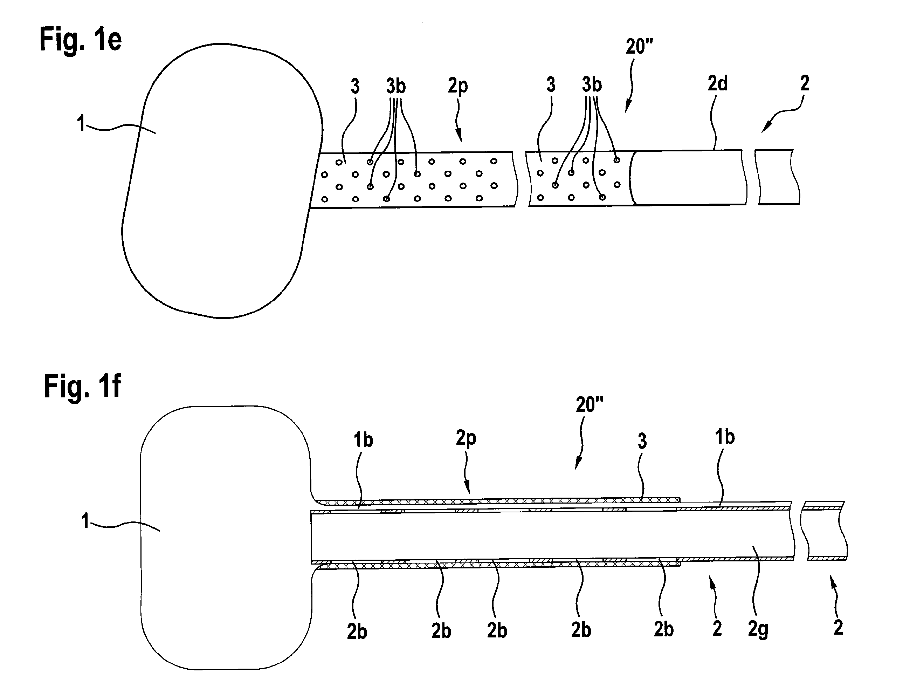

In den Figuren 1a und 1b ist eine erste Ausführungsform eines erfindungsgemäßen Katheters 20 in Form einer Ballondrainage in einer Seitenansicht (Fig. 1 a) sowie in einer Längsschnittansicht (Fig. 1 b) dargestellt.

Die Ballondrainage ist mit einem Expansionselement in Form eines expandierten Ballons 1 dargestellt, dessen Querschnitt zum Festhalten des distalen Katheterendes in einer Körperhöhle vergrößert ist. Der Ballon 1 sitzt ringförmig auf einem Fluidleitelement 2 auf, das einen Drainageschlauch 2d mit einem Innenlumen 2g aufweist, der sich von dem aus dem Körper austretenden und in den Figuren rechts dargestellten proximalen Ende in Richtung auf das im Körperinneren anzuordnende und in den Figuren links dargestellte distale Ende des Katheters erstreckt. Die Figuren zeigen im Wesentlichen nur den distalen Endabschnitt des Katheters; die Längserstreckung des Drainageschlauchs 2d ist verkürzt dargestellt.

Distal des Ballons 1 ist der Drainageschlauch 2d von einem Wandabschnitt 2c aus einem offenporigen Material 3 in Form einer offenporigen Folie mit multiplen Porenöffnungen 3b circulär ummantelt.

Der Drainageschlauch 2d weist eine distale Schlauchöffnung 2a und seitliche Perforationsöffnungen 2b auf, wobei zumindest ein Teil der seitlichen Perforationsöffnungen 2b von dem offenporigen Material 3 abgedeckt ist. Das offenporige Material 3 umläuft einen Teil des distalen Endabschnitts des Drainageschlauchs vollständig. Aus der distalen Schlauchöffnung 2a ragt ein Führungsdraht 4.

Fig. 1b ist eine Längsschnittdarstellung der ersten Ausführungsform. Man erkennt den expandierten Ballon 1, der mit einem Kanal 1 b zur Füllung des Ballons 1 fluidleitend verbunden ist. Der Kanal 1 b liegt in der Wand des Drainageschlauchs 2d. Distal des Ballons 1 ist der Drainageschlauch 2d von der offenporigen Folie ummantelt. Ferner sind die seitlichen Perforationsöffnungen 2b in der Schlauchwand dargestellt, von denen einige durch das offenporige Material 3 abgedeckt sind. Aus der distalen Öffnung 2a des Schlauches ragt der in dem Innenlumen 2g durch das Fluidleitelement 2 verlaufende Führungsdraht 4.

In den Figuren 1c und 1d ist eine zweite Ausführungsform eines erfindungsgemäßen Katheters 20' in Form einer Ballondrainage in einer Seitenansicht (Fig. 1 c) sowie in einer Längsschnittansicht (Fig. 1d) dargestellt.

Die Ballondrainage ist mit einem Expansionselement in Form eines expandierten Ballons 1 dargestellt, dessen Querschnitt zum Festhalten des distalen Katheterendes in einer Körperhöhle vergrößert ist. Der Ballon 1 sitzt ringförmig auf einem Fluidleitelement 2 auf, das einen Drainageschlauch 2d mit einem Innenlumen 2g aufweist, der sich von dem aus dem Körper austretenden und in den Figuren rechts dargestellten proximalen Ende in Richtung auf das im Körperinneren anzuordnende und in den Figuren links dargestellte distale Ende des Katheters erstreckt. Die Figuren zeigen im Wesentlichen nur den distalen Endabschnitt des Katheters; die Längserstreckung des Drainageschlauchs 2d ist verkürzt dargestellt.

Distal des Ballons 1 ist der Drainageschlauch 2d von einem ersten Wandabschnitt 2c aus einem offenporigen Material 3 in Form einer offenporigen Folie mit multiplen Porenöffnungen 3b circulär ummantelt. Proximal des Ballons 1 ist der Drainageschlauch von einem zweiten Wandabschnitt 2p aus einem offenporigen Material 3 in Form einer offenporigen Folie mit multiplen Porenöffnungen 3b circulär ummantelt.

Der Drainageschlauch 2d weist eine distale Schlauchöffnung 2a und seitliche Perforationsöffnungen 2b auf, wobei zumindest ein Teil der seitlichen Perforationsöffnungen 2b von dem offenporigen Material 3 abgedeckt ist. Das offenporige Material 3 umläuft einen Teil des distalen Endabschnitts des Drainageschlauchs 2d vollständig. Aus der distalen Schlauchöffnung 2a ragt ein Führungsdraht 4. Das offenporige Material 3 ist distal (2c) und proximal (2p) des expandierten Ballons 1 angeordnet.

Fig. 1d ist eine Längsschnittdarstellung der zweiten Ausführungsform. Man erkennt den expandierten Ballon 1, der mit einem Kanal 1b zur Füllung des Ballons 1 fluidleitend verbunden ist. Der Kanal 1 b liegt in der Wand des Drainageschlauchs 2d. Distal (2c) und proximal (2p) des Ballons 1 ist der Drainageschlauch 2d von dem offenporigen Material 3 ummantelt. Ferner sind die seitlichen Perforationsöffnungen 2b in der Schlauchwand dargestellt, von denen einige durch das offenporige Material 3 abgedeckt sind. Aus der distalen Öffnung 2a des Schlauches ragt der in dem Innenlumen 2g durch das Fluidleitelement 2 verlaufende Führungsdraht 4.

In den Figuren 1e und 1f ist eine dritte Ausführungsform eines erfindungsgemäßen Katheters 20" in Form einer Ballondrainage in einer Seitenansicht (Fig. 1 e) sowie in einer Längsschnittansicht (Fig. 1f) dargestellt.

Die Ballondrainage ist mit einem Expansionselement in Form eines expandierten Ballons 1 dargestellt, dessen Querschnitt zum Festhalten des distalen Katheterendes in einer Körperhöhle vergrößert ist. Der Ballon 1 ist am distalen Ende eines Fluidleitelements 2 angeordnet, das einen Drainageschlauch 2d mit einem Innenlumen 2g aufweist, der sich von dem aus dem Körper austretenden und in den Figuren rechts dargestellten proximalen Ende in Richtung auf das im Körperinneren anzuordnende und in den Figuren links dargestellte distale Ende des Katheters erstreckt. Die Figuren zeigen im Wesentlichen nur den distalen Endabschnitt des Katheters; die Längserstreckung des Drainageschlauchs ist verkürzt dargestellt.

Proximal des Ballons 1 ist der Drainageschlauch 2d von einem Wandabschnitt 2p aus einem offenporigen Material 3 in Form einer offenporigen Folie mit multiplen Porenöffnungen 3b circulär ummantelt.

Der Drainageschlauch 2d endet distal mit dem Ballon 1 und weist proximal des Ballons seitliche Perforationsöffnungen 2b auf, wobei zumindest ein Teil der seitlichen Perforationsöffnungen 2b von dem offenporigen Material 3 abgedeckt ist. Das offenporige Material 3 umläuft einen Teil des distalen Endabschnitts des Drainageschlauchs 2d vollständig.

Fig. 1f ist eine Längsschnittdarstellung der dritten Ausführungsform. Man erkennt den expandierten Ballon 1, der mit einem Kanal 1b zur Füllung des Ballons 1 fluidleitend verbunden ist. Der Kanal 1 b liegt in der Wand des Drainageschlauchs 2d. Proximal (2p) des Ballons 1 ist der Drainageschlauch 2d von der offenporigen Folie ummantelt. Ferner sind die seitlichen Perforationsöffnungen 2b in der Schlauchwand proximal des Ballons dargestellt, die durch das offenporige Material 3 abgedeckt sind.

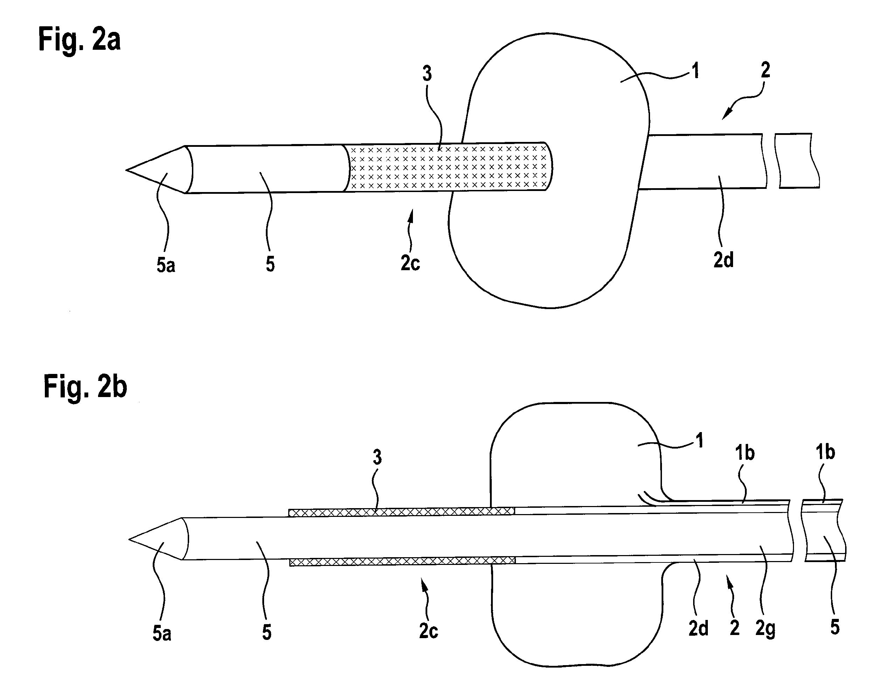

Figur 2a ist eine Seitenansicht einer vierten Ausführungsform der Erfindung in Form einer Ballondrainage. Im Unterschied zu der ersten Ausführungsform ist das Fluidleitelement 2 hier verschieblich auf einen Punktionsmandrin 5 geschoben. Die Spitze 5a des Mandrin 5a ist scharf. Der Schlauch 2d des Fluidleitelements 2 endet in einem distalen Wandabschnitt 2c aus einem offenporigen Material 3 zur Fluiddrainage. Auf dem Schlauch 2d sitzt ein aufgeblasener ringförmiger Ballon 1, der über einen Kanal 1 b aufblasbar ist.

Figur 2b ist eine Längsschnittdarstellung der vierten Ausführungsform. Man erkennt den Punktionsmandrin 5 mit scharfer Spitze 5a. Der Schlauch endet in einem offenporigen Wandabschnitt 2c. Der aufgeblasene Ballon 1 ist fluidleitend mit dem Kanal 1 b zur Füllung des Ballons 1 verbunden. Der Kanal 1 b ist in die Wand des Schlauches 2d integriert.

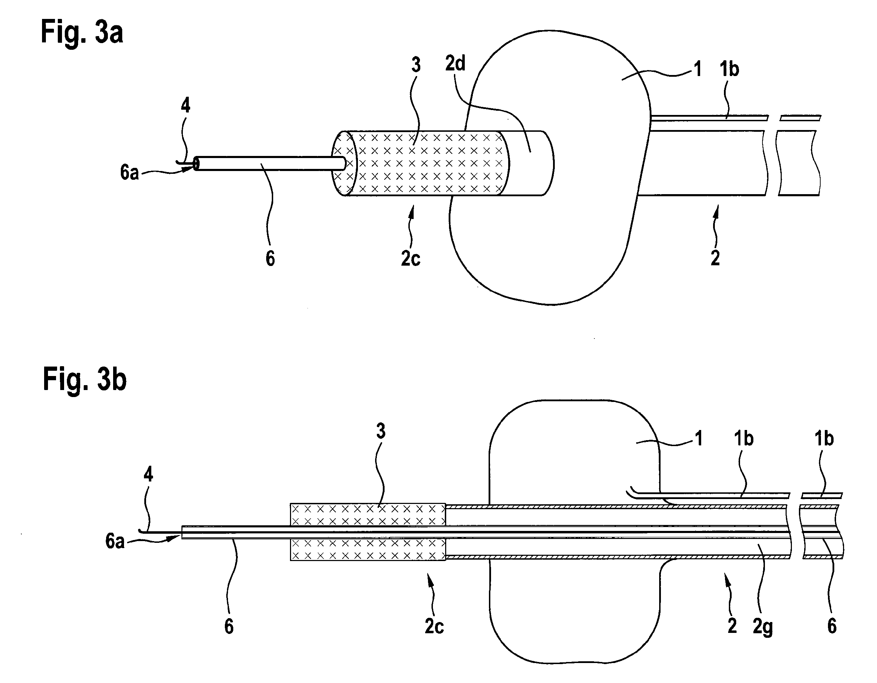

Figur 3a ist eine Seitenansicht einer fünften Ausführungsform der Erfindung in Form einer Ballondrainage. Man erkennt den expandierten Ballon 1, der mit einem Kanal 1 b in Form eines Schlauchs zur Füllung des Ballons 1 verbunden ist. Das Fluidleitelement 2 ist von dem Kanal 1 b zum Füllen des Ballons beabstandet. Der Drainageschlauch 2d des Fluidleitelements 2 endet distal in einem offenporigen Wandabschnitt 2c. In dem Innenlumen 2g des Fluidleitelements 2 ist mittig ein Spülkanal 6 in Form eines weiteren Schlauchs eingeführt, aus dessen distaler Öffnung 6a ein Führungsdraht 4 ragt. Über den Spülkanal 6 kann Flüssigkeit zugeführt werden. An das proximale Ende des Fluidleitelements 2 wird der Unterdruck angelegt.

Figur 3b ist eine Längsschnittdarstellung der fünften Ausführungsform. Der expandierte Ballon 1 ist fluidleitend mit einem Kanal 1 b verbunden, der von dem Fluidleitelement 2 beabstandet ist. In dem Innenlumen 2g verläuft der Spülkanal 6 in Form eines weiteren Schlauchs zur Spülung. Aus dem Schlauchende 6a ragt ein Führungsdraht 4.

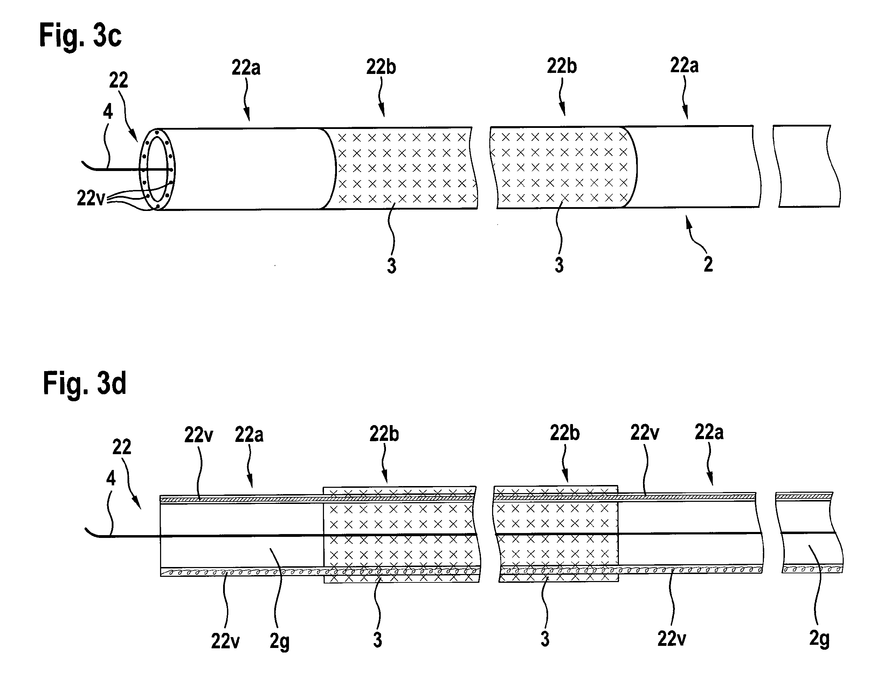

In den Figuren 3c und 3d ist eine Detaildarstellung eines Teils eines Fluidleitelements einer sechsten Ausführungsform eines erfindungsgemäßen Katheters in einer Seitenansicht (Fig. 3c) sowie in einer Längsschnittansicht (Fig. 3d) dargestellt.

Die Seitenansicht gemäß Fig. 3c zeigt das Fluidleitelement 2, das aus schlauchförmigen Abschnitten 22a und offenporigen Abschnitten 22b besteht. Der offenporige Abschnitt 22b besteht vollständig aus einem offenporigen Material 3. Aus dem proximalen Endes des Fluidleitelements 2 ragt ein Führungsdraht 4. An dem distalen Ende 22 des Schlauchs erkennt man Verstärkungselemente 22v in der Schlauchwand.

In der Längsschnittansicht gemäß Fig. 3d erkennt man die schlauchförmigen Drainageabschnitte 22a und einen offenporigen Drainageabschnitt 22b. Diese sind durch spiralförmige und gerade fadenförmige Elemente 22v wandverstärkt. Der schlauchförmige Abschnitt 22a hat ein Innenlumen 2g, das mit dem offenporigen Material 3 in Fluidverbindung steht. Im Bereich des offenporigen Abschnitts 22b besitzt das Fluidleitelement 2 selbst kein fluidleitendes kanalförmiges Innenlumen, welches in der Längsachse der Drainage verläuft. Die Fluidleitung findet entlang der offenen Poren statt. Durch das offenporige Material 3 und durch das Innenlumen 2g des schlauchförmigen Abschnitts 22a ist ein Führungsdraht 4 geführt.

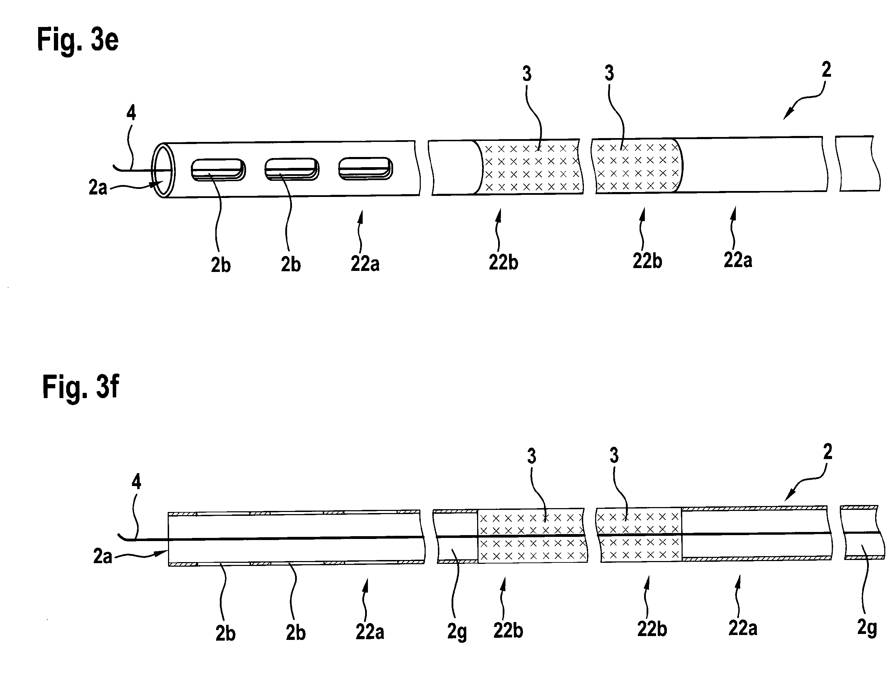

In den Figuren 3e und 3f ist eine Detaildarstellung eines Teils eines Fluidleitelements 2 einer siebten Ausführungsform eines erfindungsgemäßen Katheters in einer Seitenansicht (Fig. 3e) sowie in einer Längsschnittansicht (Fig. 3f) dargestellt.

Ein Drainageschlauch 22a weist eine distale Schlauchöffnung 2a und seitliche Perforationsöffnungen 2b auf, ein mittlerer Abschnitt des Fluidleitelements 2 besteht aus einem offenporigen Teilabschnitt 22b, welcher aus einem offenporigen Material 3 besteht. In den Katheter ist ein Führungsdraht 4 eingeführt.

Fig. 3f ist eine Längsschnittdarstellung der siebten Ausführungsform. Man erkennt den distalen Schlauchabschnitt mit seitlichen Perforationsöffnungen 2b und einem Innenlumen 2g. Der mittlere Abschnitt 22b besteht aus einem offenporigen Material 3, welches nicht mit einem Kanal oder Innenlumen längs der Hauptachse des Fluidleitelements 2 ausgerüstet ist, aber mit dem Innenlumen 2g des Drainageschlauchs 22a in Fluidverbindung steht. Die Fluidleitung findet entlang der offenen miteinander kommunizierenden Poren statt. Aus der distalen Öffnung 2a des Schlauches ragt der in dem Innenlumen 2g und durch das offenporige Material 3 verlaufende Führungsdraht 4.

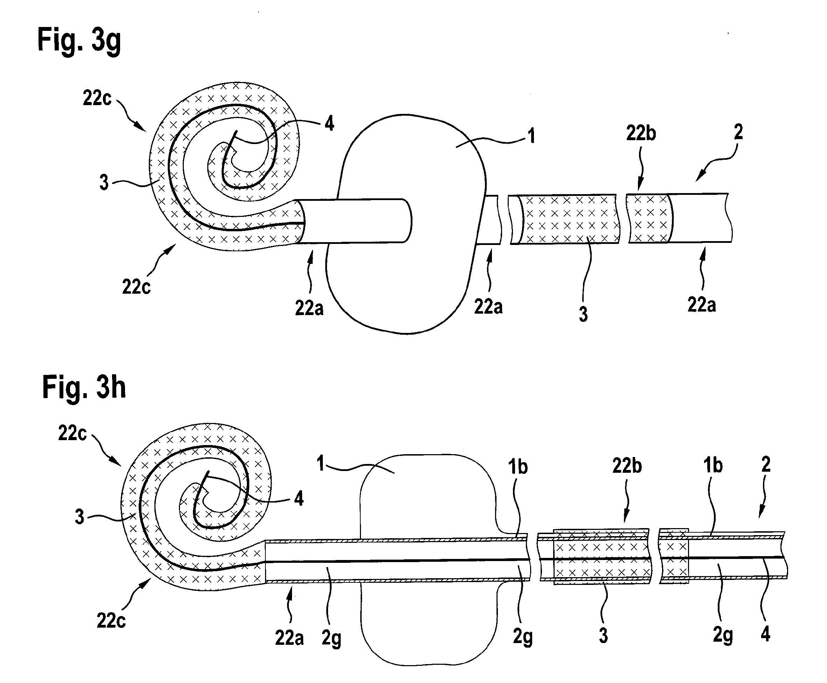

In den Figuren 3g und 3h ist eine achte Ausführungsform eines erfindungsgemäßen Katheters in Form einer Ballondrainage in einer Seitenansicht (Fig. 3g) und einer Längsschnittansicht (Fig. 3h) dargestellt. Die Drainage ist mit einem Expansionselement in Form eines expandierten Ballons 1 dargestellt, dessen Querschnitt zum Festhalten des distalen Katheters in einem Hohlorgan vergrößert ist. Der Ballon 1 sitzt ringförmig auf einem Fluidleitelement 2 auf, das einen schlauchförmigen Abschnitt 22a mit einem Innenlumen 2g aufweist, der sich von dem aus dem Körper austretenden und in den Figuren rechts dargestellten proximalen Ende in Richtung auf das im Körperinneren anzuordnende und in der Figur links dargestellte distale Ende des Katheters erstreckt. Der Ballon 1 ist über einen Kanal 1 b aufblasbar.

Das distale Ende 22c des Schlauches besteht aus einem offenporigen Material 3, es besitzt selbst kein Innenlumen, die Fluidleitung geschieht entlang der offenen Poren. Aus dem distalen Ende 22c ragt ein Führungsdraht 4. Das distale Ende 22c ist schweineschwänzchenartig geringelt.

Auch ein mittlerer Abschnitt 22b des Fluidleitelements 2 besteht aus einem offenporigen Material 3 und besitzt in diesem Abschnitt kein fluidleitendes Innenlumen entlang der Längsachse; man erkennt den Kanal 1 b zur Befüllung des Ballons 1.

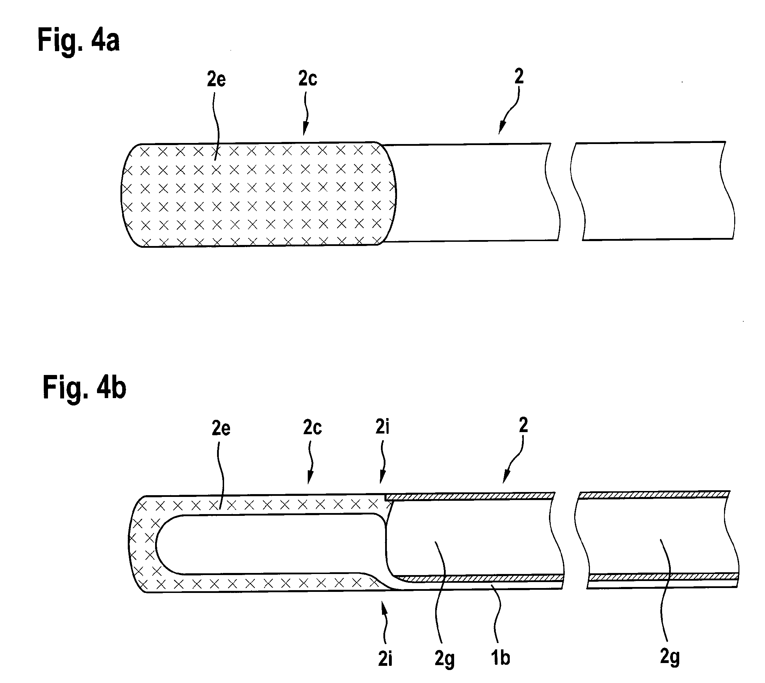

Figur 4a ist eine Seitenansicht einer neunten Ausführungsform der Erfindung in Form einer Ballondrainage. Der Ballon 1 ist bei dieser Darstellung noch nicht entfaltet, sondern kollabiert. Man erkennt das Fluidleitelement 2 mit Drainageschlauch, der an seinem distalen Ende 2e offenporig endet. Die distale Front des Katheters endet hier rundlich atraumatisch.

Figur 4b ist eine Längsschnittdarstellung der neunten Ausführungsform. In die Seitenwand des Fluidleitelements 2 ist ein Kanal 1 b integriert, der zur Füllung des Ballons 1 dient, der sich zylindrisch in dem distalen Endes 2e des Drainageschlauchs erstreckt. Die dehnbare Außenwand des kollabierten Ballon 1 ist von der offenporigen Wand 2c, welche sich elastisch mit dem Ballon 1 ausdehnen kann und dabei weiterhin offenporig fluidleitend bleibt, umhüllt. Unter einem Unterdruck werden Flüssigkeiten oder Gase durch den offenporigen Wandabschnitt 2c in das Innenlumen 2g des Schlauches geleitet. Die Wand des Drainageschlauchs geht bei 2i in den offenporigen Wandabschnitt 2c über.

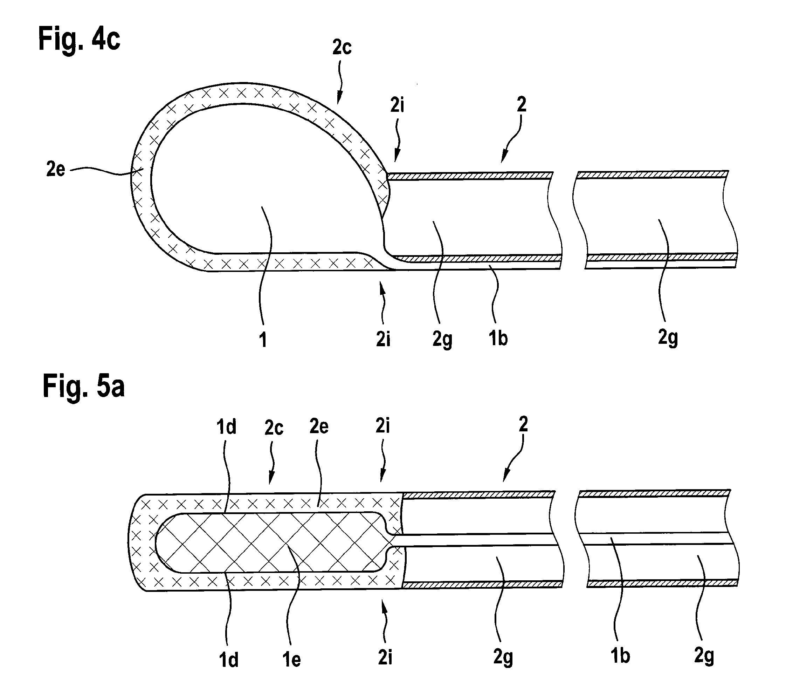

Figur 4c ist eine Längsschnittdarstellung der neunten Ausführungsform, bei der der Ballon 1 expandiert ist. In eine Seitenwand des Fluidleitelements 2 ist der Kanal 1 b integriert, der zur Füllung des Ballons 1 dient. Die offenporige Wand 2c am distalen Endes 2e des Fluidleitelements 2 dehnt sich mit dem Ballon 1 und behält dabei weiterhin die offenporige Fluidleitung für Gase und Flüssigkeiten. Unter einem Unterdruck werden Flüssigkeiten oder Gase durch die offenporige Wand 2c in das Innenlumen 2g des Schlauches geleitet.

Figur 5a ist eine Längsschnittansicht einer zehnten Ausführungsform der Erfindung in Form einer Ballondrainage. Der Schlauchabschnitt des Fluidleitelements 2 endet in distal in dem offenporigen Wandabschnitt 2c. Der offenporige Wandabschnitt 2c umschließt den Ballon 1, der mit einem elastisch komprimierbaren Füllmaterial 1e (z.B. einem offenporigen Polyurethanschaum) gefüllt ist. Die elastische Ballonwand, an der außen der Wandabschnitt 2c angeordnet ist, ist mit 1d bezeichnet. Über den Kanal 1b ist ein Unterdruck an den Ballon 1 angelegt worden, so dass das Füllmaterial 1 e zusammengezogen ist. Der Schlauchabschnitt des Fluidleitelements 2 geht bei 2i in den offenporigen Wandabschnitt 2c über. Das fluidleitende Innenlumen ist mit 2g bezeichnet.

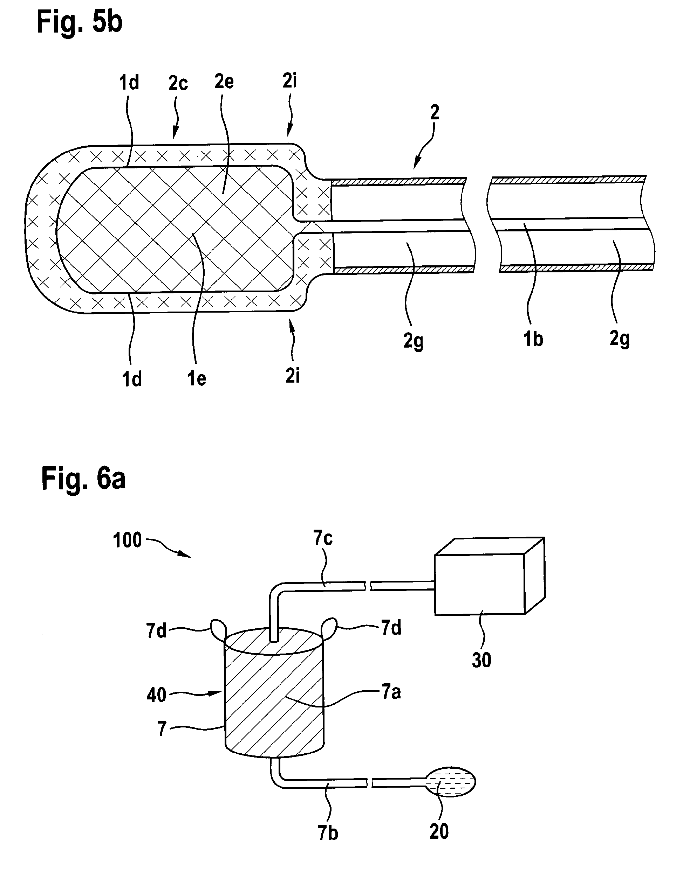

Figur 5b ist eine Längsschnittdarstellung dieser Ballondrainage. Hier ist aber an den Kanal 1 b kein Unterdruck angelegt, sondern ein normaler Luftdruck anliegend. Das komprimierbare Füllmaterial 1e hat sich daher entfaltet und mit der Ballonwand 1d und dem offenporigen Wandabschnitt 2c ausgedehnt. Auf diese Weise kann sich eine, in einen Hohlraum eingelegte Ballondrainage mit sanftem Ausdehndruck dem Hohlraum anpassen, so dass möglichst viel offenporige Drainagefläche an dem Körper anliegt und flächig gesaugt werden kann.

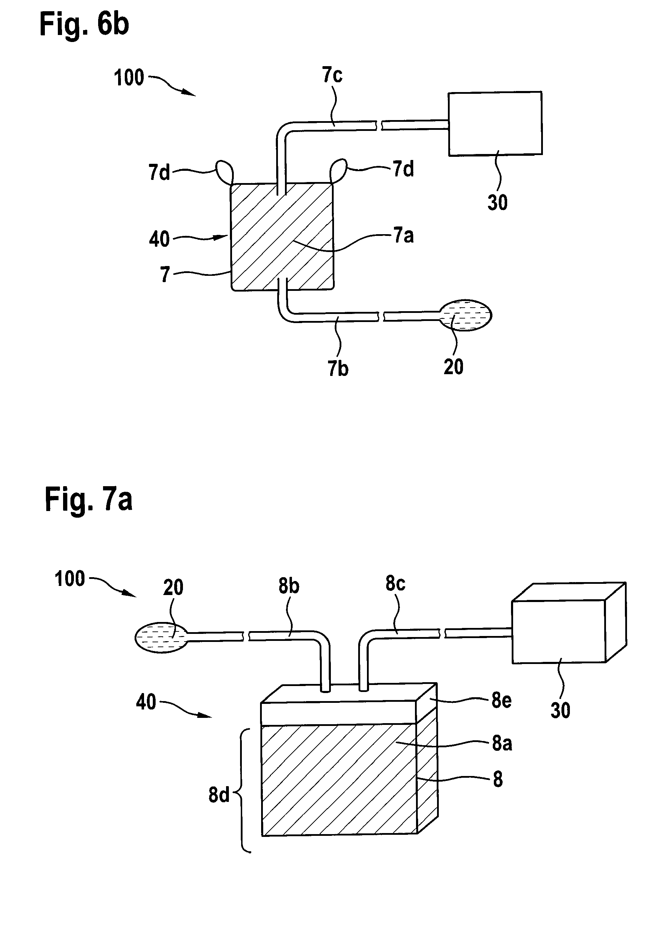

Figur 6a zeigt eine Seitenansicht eines erfindungsgemäßen Kathetersystems 100, und Fig. 6b zeigt das Kathetersystem 100 in einer Schnittdarstellung. Das Kathetersystem 100 weist auf: einen Fluidauffangbehälter 40 in Form eines Drainagebeutels 7, ein unterdruckerzeugendes System 30 in Form einer elektronischen Pumpe und einen erfindungsgemäßen Katheter 20 (in der Zeichnung nur angedeutet). Der Drainagebeutel 7 ist mit einem komprimierbaren offenporigen Füllmaterial 7a gefüllt. Über einen ersten Schlauch 7b ist der Beutel 7 mit dem Katheter 20 verbunden, über einen zweiten Schlauch 7c ist der Beutel 7 mit der Pumpe verbunden. Der Beutel 7 kann mit zwei Schlaufen 7d aufgehängt werden.

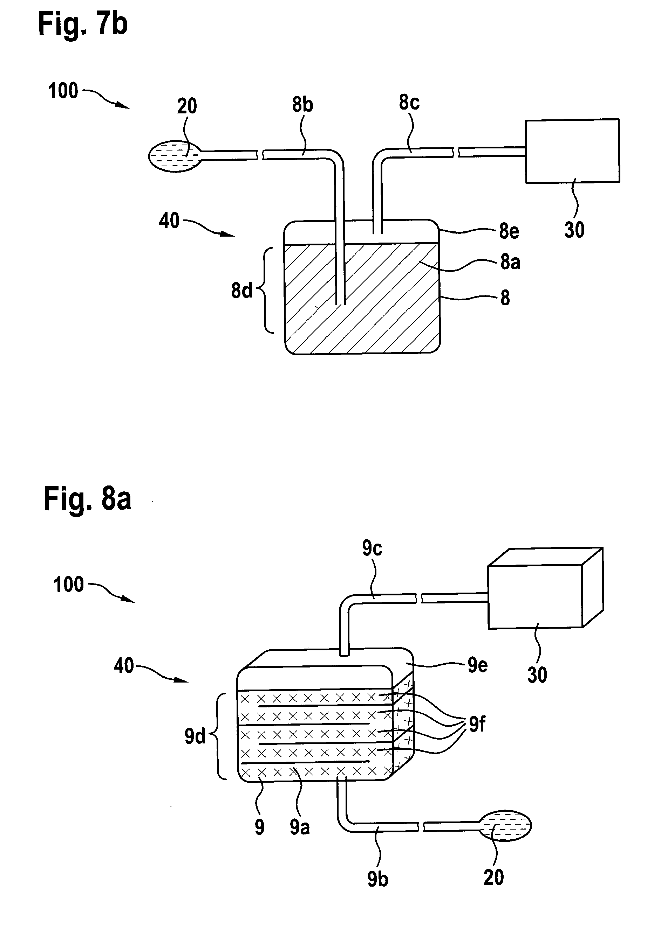

Figur 7a zeigt eine Seitenansicht einer alternativen Ausführungsform eines erfindungsgemäßen Kathetersystems, und Fig. 7b zeigt dieses Kathetersystem in einer Schnittdarstellung. Das Kathetersystem weist auf: einen Fluidauffangbehälter 40, ein unterdruckerzeugendes System 30 in Form einer elektronischen Vakuumpumpe und einen erfindungsgemäßen Katheter 20. Der Fluidauffangbehälter 40 ist in Form eines Sekretauffangbehältnisses 8 gebildet, welches aus einem festen Kanisterteil 8e und einem flexiblen Beutelteil 8d besteht. In dem Beutelteil 8d befindet sich ein offenporiges komprimierbares Füllmaterial 8a wie etwa ein offenporiger Polyurethanschaum. Über einen ersten Schlauch 8b ist das Sekretauffangbehältnis 8 mit dem Katheter 20 verbunden. Mit einem zweiten Schlauch 8c ist das Sekretauffangbehältnis 8 mit der elektronischen Vakuumpumpe 30 verbunden. In Fig. 7b ist gezeigt, dass der erste Schlauch 8b bis tief in das Füllmaterial 8a hineinreicht, während der zweite Schlauch 8c nur in den festwandigen unterdruckstabilen Kanisterteil 8e reicht.

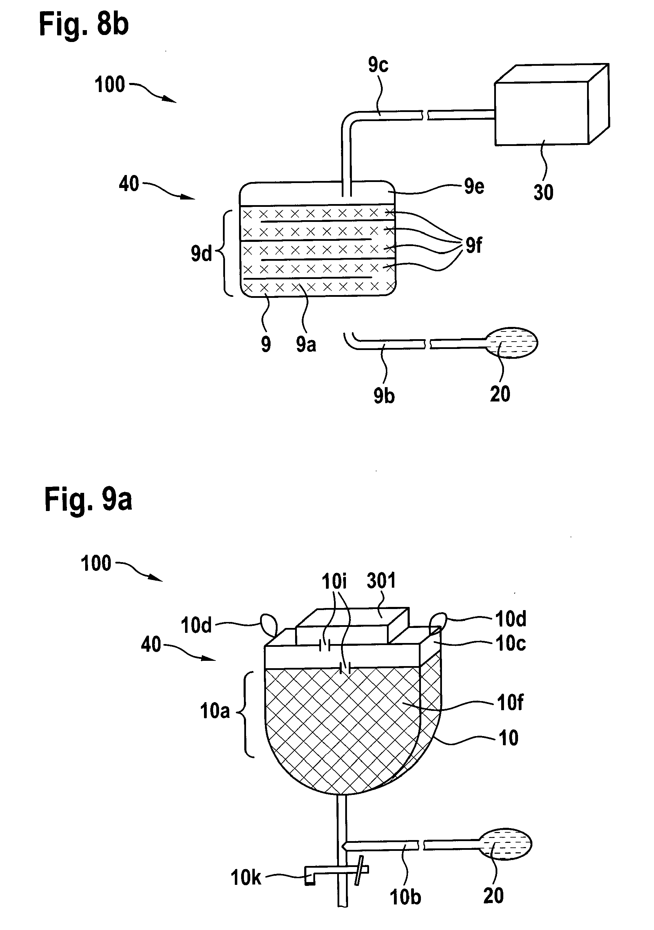

Figur 8a zeigt eine Seitenansicht einer weiteren alternativen Ausführungsform eines erfindungsgemäßen Kathetersystems, und Fig. 8b zeigt dieses Kathetersystem in einer Schnittdarstellung. Das Kathetersystem weist auf: einen Fluidauffangbehälter 40, ein unterdruckerzeugendes System 30 in Form einer elektronischen Vakuumpumpe und einen erfindungsgemäßen Katheter 20. Der Fluidauffangbehälter 40 ist in Form eines Sekretauffangbehältnisses 9 gebildet, welches aus einem festen Kanisterteil 9e und einem flexiblen Beutelteil 9d besteht. In dem Beutelteil 9d befindet sich ein offenporiges komprimierbares Füllmaterial 9a wie etwa ein offenporiger Polyurethanschaum. Der Beutelteil 9d ist in einzelne Kammern 9f aufgeteilt, die mäanderförmig miteinander in Fluidverbindung stehen. Über einen ersten Schlauch 9b ist das Sekretauffangbehältnis 9 mit dem Katheter 20 verbunden. Mit einem zweiten Schlauch 9c ist das Sekretauffangbehältnis 9 mit der Vakuumpumpe 30 verbunden.

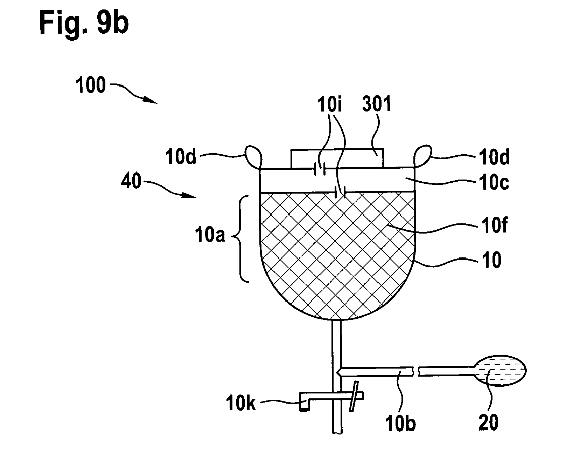

Figur 9a zeigt eine Seitenansicht einer weiteren alternativen Ausführungsform eines erfindungsgemäßen Kathetersystems, und Fig. 9b zeigt dieses Kathetersystem in einer Schnittdarstellung. Das Kathetersystem weist auf: einen Fluidauffangbehälter 40, ein unterdruckerzeugendes System in Form einer Vakuumpumpe 301 und einen erfindungsgemäßen Katheter 20. Der Fluidauffangbehälter 40 ist in Form eines Sekretauffangbehältnisses 10 gebildet, welches aus einem festen Kanisterteil 10c und einem flexiblen Beutelteil 10a besteht. In dem Beutelteil 10a befindet sich ein offenporiges komprimierbares Füllmaterial 10f wie etwa ein offenporiger Polyurethanschaum. Über einen ersten Schlauch 10b ist das Sekretauffangbehältnis 10 mit dem Katheter 20 verbunden. Die Vakuumpumpe 301 ist fluidleitend als ein integrierter abnehmbarer Teil des Sekretauffangbehältnisses 10 mit demselben lösbar verriegelt verbunden. Die Fluidleitung bzw. der Unterdruck wird über Ventile 10i von der Pumpe 301 über den Kanisterteil 10c geleitet. Mit Bezugszeichen 10k ist ein Ablaufstutzen mit Hahn gekennzeichnet; wenn dieser geöffnet wird, kann das im Sekretauffangbehältnis 10 gesammelte Sekret ablaufen.

Jedes der in den Figuren 6 bis 10 gezeigte Katheter-System kann mit einem beliebigen erfindungsgemäßen Katheter ausgestattet sein, insbesondere mit einem der Ballonkatheter, die in den Figuren 1 bis 5 dargestellt sind. Der erfindungsgemäße Katheter ist aber nicht auf einen Ballonkatheter beschränkt, sondern kann anstelle des Ballons auch ein anderes Expansionselement aufweisen. Wichtig ist, dass das Fluidleitelement des Katheters einen oder mehrere Wandabschnitte aus einem offenporigen Material wie etwa einem Schaum oder einer offenporigen Folienanordnung aufweist, wobei ein Unterdruck an das Innenlumen des Fluidleitelements anlegbar ist.

Die Erfindung ist nicht auf die oben im Detail beschriebenen Ausführungsformen beschränkt, wie sie beispielhaft in den Figuren dargestellt sind, und die in der vorliegenden Beschreibung erläuterten Merkmale können einzeln oder in beliebiger Kombination bei einem Katheter verwendet werden. Ferner wird ein erfindungsgemäßer Katheter zwar vorzugsweise, aber nicht notwendigerweise zur Unterdrucktherapie eingesetzt. Entsprechend können Ausführungsformen eines erfindungsgemäßen Katheters auch ohne ein unterdruckerzeugendes System eingesetzt werden.

## Patentansprüche

1. Katheter (20), insbesondere Ballonkatheter,
mit einem Fluidleitelement (2) mit einem Innenlumen (2g) zum Abführen von Körperflüssigkeiten und/oder Gasen aus dem Körperinneren und
mit einem Expansionselement wie etwa einem Ballon (1) mit veränderlichem Querschnitt zum Halten eines Katheterabschnitts in einem Körperhohlraum, **dadurch gekennzeichnet, dass**
das Fluidleitelement (2) mindestens einen Wandabschnitt (2c) aus einem offenporigen Material (3) aufweist, das mit dem Innenlumen (2g) in Fluidverbindung steht und durch das die Körperflüssigkeit durch Anlage eines Unterdrucks an ein proximales Ende des Fluidleitelements (2) in das Innenlumen (2g) saugbar ist.

2. Katheter nach Anspruch 1, **dadurch gekennzeichnet, dass** das offenporige Material (3) eine Vielzahl von untereinander fluidleitend kommunizierenden Poren (3b) aufweist.

3. Katheter nach Anspruch 2, **dadurch gekennzeichnet, dass** die Poren (3b) einen Durchmesser zwischen 100 µm und 2000 µm, insbesondere zwischen 100 µm und 500 µm aufweisen, und/oder dass der mittlere Abstand zwischen benachbarten Poren (3b) zwischen 100 µm und 5000 µm, insbesondere zwischen 100 µm und 1000 µm liegt.

4. Katheter nach einem der vorhergehenden Ansprüche, **dadurch gekennzeichnet, dass** das offenporige Material (3) in Form eines offenporigen Schaums wie etwa eines Polyurethanschaums oder in Form einer offenporigen einlagigen, doppellagigen oder mehrlagigen Folie ausgebildet ist.

5. Katheter nach einem der vorhergehenden Ansprüche, **dadurch gekennzeichnet, dass** das Innenlumen (2g) zumindest abschnittsweise unmittelbar von dem offenporigen Material (3) begrenzt ist, oder alternativ dass das Innenlumen (2g) von einer Öffnungen (2b) aufweisenden Seitenwand bewandet ist, wobei die Öffnungen (2b) zumindest teilweise außen von dem offenporigen Material (3) abgedeckt sind.

6. Katheter nach einem der vorhergehenden Ansprüche, **dadurch gekennzeichnet, dass** das offenporige Material (3) einseitig offenporig ist, wobei die offenporige Seite zur Anlage an einem Körpergewebe nach außen gewandt ist und die der offenporigen Seite entgegengesetzte innere Seite dem Innenlumen (2g) zugewandt ist und Durchlässe aufweist.

7. Katheter nach einem der vorhergehenden Ansprüche, **dadurch gekennzeichnet, dass** das offenporige Material (3) eine nichttransparente, insbesondere eine farbige Folie aufweist.

8. Katheter nach einem der vorhergehenden Ansprüche, **dadurch gekennzeichnet, dass** der Wandabschnitt (2c) aus offenporigem Material (3) in einem distalen Endabschnitt des Fluidleitelements (2) angeordnet ist.

9. Katheter nach einem der vorhergehenden Ansprüche, **dadurch gekennzeichnet, dass** das Expansionselement ein Ballon (1) mit einer elastisch dehnbaren Wand ist, die zumindest teilweise ein Füllvolumen des Ballons bewandet.

10. Katheter nach Anspruch 9, **dadurch gekennzeichnet, dass** das Füllvolumen des Ballons (1) zumindest teilweise von einem Füllmaterial wie etwa einem offenporigen Polyurethanschaum gefüllt ist, das sich bei Anlegen eines Unterdrucks an das Füllvolumen zusammenzieht und das bei Aufheben des Unterdrucks eine Entfaltung des Ballons bewirkt.

11. Katheter nach Anspruch 9 oder 10, **gekennzeichnet durch** einen fluid- und/oder gasleitenden Kanal (1b) zum Befüllen oder Entleeren des Füllvolumens, der in oder im Wesentlichen parallel zu dem Fluidleitelement (2) verläuft.

12. Katheter nach Anspruch 11, **dadurch gekennzeichnet, dass** der Kanal (1 b) zumindest abschnittsweise im Innenlumen (2g) des Fluidleitelements, außerhalb des Innenlumens (2g) des Fluidleitelements, in einer oder anliegend an eine Seitenwand des Fluidleitelements (2) in dessen Längsrichtung oder alternativ als separater Schlauch beabstandet von dem Fluidleitelement (2) verläuft.

13. Katheter nach einem der Ansprüche 9 bis 12, **dadurch gekennzeichnet, dass** sich der Wandabschnitt (2c) aus dem offenporigen Material (3) distal an den Ballon (1) anschließt oder alternativ dass der Ballon (1) das distale Ende des Katheters bildet und der Wandabschnitt (2c) aus dem offenporigen Material zumindest abschnittsweise außen an der elastisch dehnbaren Wand des Ballons (1) angeordnet ist.

14. Katheter nach einem der vorhergehenden Ansprüche, **dadurch gekennzeichnet, dass** ein distales Ende des Katheters (20) geschlossen ausgebildet ist und ein Befestigungselement wie etwa eine Öse, einen Faden, eine Kugel o.dgl. zum Anbringen eines Greif- oder Zugelements aufweist.

15. Katheter nach einem der Ansprüche 1 bis 13, **dadurch gekennzeichnet, dass** ein distales Ende des Katheters eine Öffnung (2a) aufweist, durch die ein Führungsdraht (4) oder Führungsmandrin (5) einführbar ist.

16. Katheter nach einem der vorhergehenden Ansprüche, **dadurch gekennzeichnet, dass** ein distales Ende des Katheters (20) eine Schneide oder Spitze (5a) zum Punktieren von Gewebe aufweist.

17. Katheter nach einem der vorhergehenden Ansprüche, **dadurch gekennzeichnet, dass** der Katheter (20) zumindest einen an seinem distalen Ende eine Öffnung (6a) aufweisenden Spülkanal (6) zum Spülen oder zum Zuführen von Flüssigkeiten, Medikamenten oder Messinstrumenten zu einem Körperhohlraum aufweist.

18. Katheter-System (100) mit einem Katheter (20) nach einem der vorhergehenden Ansprüche und einem unterdruckerzeugenden System (30) wie etwa einem Pumpsystem, das zum Erzeugen eines Sogs in dem Innenlumen (2g) des Fluidleitelements (2) an das proximale Ende des Fluidleitelements (2) anschließbar ist.

19. Katheter-System nach einem der vorhergehenden Ansprüche, **gekennzeichnet durch** einen zwischen das unterdruckerzeugende System (30) und das Fluidleitelement (2) geschalteten unterdruckstabilen Fluidauffangbehälter (40) wie etwa ein Beutel- und/oder Kanistersystem.

20. Katheter-System nach einem der vorhergehenden Ansprüche, **dadurch gekennzeichnet, dass** das unterdruckerzeugende System (30) zur Erzeugung eines permanenten Unterdrucks zwischen etwa 40 mmHg und etwa 100 mmHg, insbesondere zwischen etwa 20 mmHg und etwa 200 mmHg eingerichtet ist.
